# EUROPEAN PATENT SPECIFICATION

(11) **EP 1 789 107 B1**
(45) Date of publication and mention of the grant of the patent: **27.05.2009**
(21) Application number: 05792356.7
(22) Date of filing: 30.08.2005
(51) Int. Cl.: A61L 31/16, A61L 31/08, A61K 31/19, A61K 31/352, A61K 45/00, A61K 45/06, A61K 31/70, A61K 31/197, A61K 9/00, A61P 35/00

(54) **MEDICAL STENT PROVIDED WITH INHIBITORS OF ATP SYNTHESIS**
MEDIZINISCHER STENT MIT ATP-SYNTHESEHEMMERN
ENDOPROTHESE MEDICALE POURVUE D'INHIBITEURS DE LA SYNTHESE D'ATP

(30) Priority: 30.08.2004 WO PCT/EP2004/009639; 15.12.2004 US 635778 P; 15.12.2004 EP 04447279
(43) Date of publication of application: 30.05.2007
(73) Proprietor: Interstitial Therapeutics, 1206 Geneva (CH)
(72) Inventor: POPOWSKI, Youri, CH-1206 Geneva (CH)
(74) Representative: De Clercq, Ann G. Y.
(86) International application number: PCT/EP2005/009323
(87) International publication number: WO 2006/024488

(56) References cited:
- EP-A- 1 407 786
- WO-A-01/82926
- WO-A-99/18798
- WO-A-03/072024
- WO-A-20/04062604
- WO-A-20/04064734
- US-A1- 2003 018 353
- US-A1- 2003 096 870
- US-A1- 2004 086 550

## Description

### FIELD OF THE INVENTION

The present invention relates to a stent useful for expanding a duct lumen or resectioned cavity or scar in a subject and inhibiting cell proliferation in the vicinity of the stent.

### BACKGROUND TO THE INVENTION

A stent is commonly used as a tubular structure introduced inside the lumen of a duct to relieve an obstruction. Commonly, stents are inserted into the lumen of the duct in a non-expanded form and are then expanded autonomously (or with the aid of a second device) *in situ*.

When a stent is used to expand a vascular lumen, restenosis (re-narrowing) may occur. Restenosis of an artherosclerotic coronary artery after a stand-alone angioplasty may occur in 10-50% of patients within 6 months, requiring either further angioplasty or coronary artery bypass graft. It is presently understood that the process of fitting a bare stent (without any drug), besides opening the artherosclerotically obstructed artery, also injures resident coronary arterial smooth muscle cells (SMC). In response to this trauma, adhering platelets, infiltrating macrophages, leukocytes, or the smooth muscle cells (SMC) themselves release cell derived growth factors with subsequent proliferation and migration of medial SMC through the internal elastic lamina to the area of the vessel intima. Further proliferation and hyperplasia of intimal SMC and, most significantly, production of large amounts of extracellular matrix over a period of 3-6 months results in the filling in and narrowing of the vascular space sufficient to significantly obstruct coronary blood flow.

To reduce or prevent restenosis, stents are provided with a means for delivering an inhibitor of SMC proliferation directly to the wall of the expanded vessel. Such delivery means include, for example, via the struts of a stent, a stent graft, grafts, stent cover or sheath, composition with polymers (both degradable and nondegrading) to hold the drug to the stent or graft or entrapping the drug into the metal of the stent or graft body which has been modified to contain micropores or channels. Other delivery means include covalent binding of the drug to the stent via solution chemistry techniques (such as via the Carmeda process) or dry chemistry techniques (e.g. vapour deposition methods such as rf-plasma polymerization) and combinations thereof. Examples of some means for delivery are mentioned in patent document US 6,599,314.

Inhibitors of SMC proliferation include sirolimus (or rapamycin,an immunosuppressive agent) and paclitaxel (or taxol, an antiproliferative, anti-angiogenic agent) . Other agents which have demonstrated the ability to reduce myointimal thickening in animal models of balloon vascular injury are heparin, angiopeptin (a somatostatin analog), calcium channel blockers, angiotensin converting enzyme inhibitors (captopril, cilazapril), cyclosporin A, trapidil (an antianginal, antiplatelet agent), terbinafine (antifungal), colchicine (antitubulin antiproliferative), and c-myc and c-myb antisense oligonucleotides.

The problem with inhibitors of the art is that they are not always rapidly and selectively absorbed by proliferating cells. This problem would require high doses of inhibitor in order to provide an efficacious response, particularly where larger lesions are treated, such as in peripheral vessels. Furthermore, inhibitors of the art do not always have a high activity once in the cell, so requiring even higher doses to be present on the stent.

In view of the prior art, there is a need for a new type of inhibitor of stenosis and restenosis, delivered via a stent.

Cancerous cells may also proliferate within ducts, such as the vascular system, bronchial ducts, biliary ducts, oesophagus, digestive tract, urethral duct, and uretheral duct as a result of conditions other than stenosis or restenosis. For example, a tumour present on a tissue outside a duct may grow to an extent that the tumour contacts the outer surface of the duct and invade. Present treatments require the delivery of anti-cancer agents intravenously, so leading to acute toxic side effects and discomfort to the patient.

There is a need for a new method and means for treating conditions arising from rapidly proliferating cells, such as cancer, inside ducts where it provokes symptoms and reduces quality of life.

### SUMMARY OF SOME EMBODIMENTS OF THE INVENTION

The present invention relates to a medical stent provided with a composition comprising at least one type of inhibitor of ATP synthesis.

One embodiment of the invention is a medical stent provided with a composition comprising an inhibitor of oxidative phosphorylation that is rhodamine 123.

Another embodiment of the invention is a kit comprising a) at least one medical stent and b) a composition comprising an inhibitor of oxidative phosphorylation that is rhodamine 123.

Another embodiment of the invention is a use of a composition comprising an inhibitor of oxidative phosphorylation that is rhodamine 123, for the preparation of a composition for providing a medical stent for inhibiting cell proliferation via inhibition of the oxidative phosphorylation pathway.

Another embodiment of the invention is a use of a composition comprising an inhibitor of oxidative phosphorylation that is rhodamine 123, for the preparation of a composition for providing a medical stent for sensitizing proliferating cells to treatment by radiotherapy.

Another embodiment of the invention is a use of a composition comprising an inhibitor of oxidative phosphorylation that is rhodamine 123, for the preparation of a composition for providing a medical stent for sensitizing proliferating cells to treatment by chemotherapy.

Another embodiment of the invention is a stent, use or kit as described above, wherein said stent is provided with one or more cavities for containing and releasing said composition.

Another embodiment of the invention is a stent, use or kit as described above, wherein said stent is at least partly made from a material which is biodegradable *in situ.*

Another embodiment of the invention is a stent, use or kit as described above, wherein said stent comprises a magnesium based alloy.

Another embodiment of the invention is a stent, use or kit as described above, wherein said stent is at least partly made from a material which is non-biodegradable *in situ* and is impregnated with said composition.

Another embodiment of the invention is a stent, use or kit as described above, wherein said stent is at least partly provided with said composition.

Another embodiment of the invention is a stent, use or kit as described above, wherein said composition further comprises one or more slow release agents to facilitate slow release of inhibitor.

Another embodiment of the invention is a stent, use or kit as described above, wherein said slow release agent is any of magnesium alloys, poly(glycolic) acid, poly(lactic acid) or in general glycolic- and lactic acid based polymers, copolymers, poly caprolactones and in general, poly hydroxyl alkanoate,s poly(hydroxy alcanoic acids), Poly (ethylene glycol), poly vinyl alcohol, poly (orthoesters), poly (anhydrides), poly (carbonates), poly amides, poly imides, poly imines, poly (imino carbonates), poly (ethylene imines), polydioxanes, poly oxyethylene (poly ethylene oxide), poly (phosphazenes), poly sulphones, lipids, poly acrylic acids, poly methylmethacrylate, poly acryl amides, poly acrylo nitriles (Poly cyano acrylates), poly HEMA, poly urethanes, poly olefins, poly styrene, poly terephthalates, poly ethylenes, poly propylenes, poly ether ketones, poly vinylchlorides, poly fluorides, silicones, poly silicates (bioactive glass), siloxanes (Poly dimethyl siloxanes), hydroxyapatites, lactide-capronolactone, natural and non natural poly aminoacids , poly β-aminoesters, albumines, alginates, cellulose / cellulose acetates, chitin / chitosan, collagene, fibrine / fibrinogen, gelatine, lignine, proteine based polymers, Poly (lysine), poly (glutamate), poly (malonates), poly (hyaluronic acids), Poly nucleic acids, poly saccharides, poly (hydroxyalkanoates), poly isoprenoids, starch based polymers, copolymers thereof, linear, branched, hyperbranched, dendrimers, crosslinked, functionalised derivatives thereof, or hydrogels based on activated polyethyleneglycols combined with alkaline hydrolyzed animal or vegetal proteins.

Another embodiment of the invention is a stent, use or kit as described above, wherein at least one inhibitor is encapsulated in a micro-capsule or nano-capsule.

Another embodiment of the invention is a stent, use or kit as described above wherein a nano-capsule comprises any of a copolymer poly(ethylene oxide) with poly(L-Lactic acid) or with poly(beta-benzyl-L-aspartate); copolymer with poly(lactide-co-glycolide)-[(propylene oxide)-poly(ethylene oxide)]; polyphosphazene derivatives; a poly(ethylene glycol) coated nanosphere; a poly(isobutylcyanoacrylate) nanocapsules poly(gamma-benzyl-L-glutamate)/(poly(ethylene oxide); chitosan-poly(ethylene oxide) nanoparticules; o-carboxymethylate chitosan, or a solid lipid nanosphere (SLN).

Another embodiment of the invention is a stent, use or kit as described above wherein a micro-capsule is a micro-capsule comprises any of chitosan; a coated alginate microsphere; an N-(aminoalkyl) chitosan microsphere; a chitosan/calcium alginate bead, a poly(adipic anhydride) microsphere; a gellan-gum bead; a poly(D, L-lactide-co-glycolide) microsphere; an alginate-poly-L-lysine microcapsule; a crosslinked chitosan microsphere; a chitosan/gelatin microsphere; a crosslinked chitosan network bead with spacer groups; an aliphatic polyester; a 1,5-diozepan-2-one microsphere; a D,L-dilactide microsphere; a triglyceride liposphere; a polyelectrolyte complexe of sodium alginate chitosan; a polypeptide microcapsule, oran albumin microsphere.

Another embodiment of the invention is a stent, use or kit as described above, said rhodamine 123 is coupled to solubilising agent.

Another embodiment of the invention is a stent, use or kit as described above, wherein said solubilising agent is a solubilizing agent is cholesterol or derivative thereof.

Another embodiment of the invention is a stent, use or kit as described above, wherein said cholesterol derivatives are any of cholesteryl-3-betahydroxybutyrate, cholesteryl-halogenated butyrate, cholesteryl-halogenated acetate, cholesteryl-halogenated aceto-acetate, cholesteryl-halogenated acetamide, cholesteryl-halogenated crotonate, cholesteryl-halogenated acetone, cholesteryl-halogenated citrate, or cholesteryl-halogenated oleate.

Another embodiment of the invention is a stent, use or kit as described above, wherein said solubilising agent is vitamin A or derivative thereof.

Another embodiment of the invention is a stent, use or kit as described above, wherein derivative of vitamin A is formula (IV) or (V): 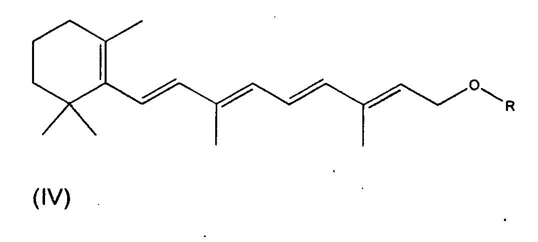 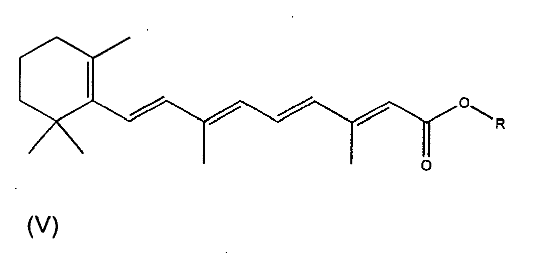 wherein R is selected from the group consisting of betahydroxybutyrate, halogenated butyrate, halogenated acetate, halogenated aceto-acetate, halogenated acetamide, halogenated crotonate, halogenated acetone, halogenated citrate, and halogenated oleate.

Another embodiment of the invention is a stent, use or kit as described above wherein the rhodamine 123 is present in a quantity to inhibit the oxidative phosphorylation pathway.

Another embodiment of the invention is a stent, use or kit as described above wherein said stent is provided with an amount of composition to deliver a concentration between 10 and 60 mg rhodamine 123 / kg.

Another embodiment of the invention is a stent, use or as described above wherein said composition further comprises one or more polymers to facilitate attachment of the composition to the stent and/or slow release of the composition.

Another embodiment of the invention is a stent, use or kit as described above, wherein said polymer is one or more of:
- aliphatic polyesters, poly(amino acids), copoly(ether-esters), polyalkylenes oxalates, polyamides, poly(iminocarbonates), polyanhydrides, polyorthoesters, polyoxaesters, polyamidoesters, polylactic acid, polyethylene oxide, polycaprolactone, polyhydroxybutyrate valerates, polyoxaesters containing amido groups, poly(anhydrides), polyphosphazenes, silicones, biomolecules and blends thereof,
- lactic acid D-,L- and meso lactide, epsilon -caprolactone, glycolide glycolic acid, hydroxybutyrate, hydroxyvalerate, para-dioxanone, trimethylene carbonate and its alkyl derivatives, 1,4-dioxepan-2-one, 1,5-dioxepan-2-one, 6,6-dimethyl-1,4-dioxan-2-one and polymer blends thereof,
- polyanhydrides from diacids of the form HOOC-C₆H₄-O-(CH₂)m-O-C₆H₄-COOH wherein m is an integer in the range of from 2 to 8 and copolymers thereof with aliphatic alpha-omega diacids of up to 12 carbons,
- naturally enzymatically or are hydrolytically unstable polymers, fibrin, fibrinogen, collagen, gelatin, glycosaminoglycans, elastin, and absorbable biocompatible polysaccharides such as chitosan, starch, fatty acids and esters thereof, glucoso-glycans and hyaluronic acid,
- hydrogels
- polyurethanes, silicones, poly(meth)acrylates, polyesters, polyalkyl oxides, polyethylene oxide, polyvinyl alcohols, polyethylene glycols and polyvinyl pyrrolidone, as well as, hydrogels, hydrogels from crosslinked polyvinyl pyrrolidinone and polyesters, polyolefins, polyisobutylene and ethylene-alphaolefin copolymers, acrylic polymers, methacrylate and copolymers, vinyl halide polymers and copolymers, polyvinyl chloride, polyvinyl ethers, polyvinyl methyl ether, polyvinylidene polyvinylidene fluoride polyvinylidene chloride, polyacrylonitrile, polyvinyl ketones, polyvinyl aromatics, polystyrene, polyvinyl esters, polyvinyl acetate, copolymers of vinyl monomers with each other and olefins, etheylene-methyl methacrylate copolymers, acrylonitrile-styrene copolymers, ABS resins and ethylene-vinyl acetate copolymers, polyamides, Nylon 66 and polycaprolactam, alkyd resins, polycarbonates, polyoxymethylenes, polyimides, polyethers, epoxy resins, polyurethanes, rayon, rayon-triacetate, cellulose, cellulose acetate, cellulose acetate butyrate, cellophane, cellulose nitrate, cellulose propionate, cellulose ethers, carboxymethyl cellulose and hydoxyalkyl celluloses, and combinations thereof,
- polyamides of the form-NH-(CH₂)n-CO- and NH-(CH₂)x-NH-CO-(CH₂)y-CO, wherein n is preferably an integer in from 6 to 13; x is an integer in the range of form 6 to 12; and y is an integer in the range of from 4 to 16,
- bioabsorbable elastomers, aliphatic polyester elastomers,
- elastomeric copolymers of epsilon-caprolactone and glycolide, preferably having a mole ratio of epsilon -caprolactone to glycolide of from about 35:65 to about 65:35, more preferably 45:55 to 35:65,
- elastomeric copolymers of epsilon-caprolactone and lactide, L-lactide, D-lactide blends thereof or lactic acid copolymers, preferably having a mole ratio of epsilon -caprolactone to lactide of from about 35:65 to about 90:10 and more preferably from about 35:65 to about 65:35 and most preferably from about 45:55 to 30:70 or from about 90:10 to about 80:20,
- elastomeric copolymers of p-dioxanone and lactide including L-lactide, D-lactide and lactic acid, preferably having a mole ratio of p-dioxanone to lactide of from about 40:60 to about 60:40,
- elastomeric copolymers of epsilon-caprolactone and p-dioxanone, preferably having a mole ratio of epsilon-caprolactone to p-dioxanone of from about 30:70 to about 70:30,
- elastomeric copolymers of p-dioxanone and trimethylene carbonate, preferably having a mole ratio of p-dioxanone to trimethylene carbonate of from about 30:70 to about 70:30,
- elastomeric copolymers of trimethylene carbonate and glycolide, preferably having a mole ratio of trimethylene carbonate to glycolide of from about 30:70 to about 70:30,
- elastomeric copolymer of trimethylene carbonate and lactide including L-lactide, D-lactide, blends thereof or lactic acid copolymers, preferably having a mole ratio of trimethylene carbonate to lactide of from about 30:70 to about 70:30 and blends thereof.

Another embodiment of the invention is a stent, or kit as described above suitable for use in inhibiting cell proliferation.

Another embodiment of the invention is a stent, use or kit as described above, wherein said cell proliferation is restenosis or stenosis.

Another embodiment of the invention is a stent, use or kit as described above, wherein said cell proliferation is cancer.

Another embodiment of the invention is a stent, use or kit as described above wherein the stent is placed in an artery or vein.

Another embodiment of the invention is a stent, use or kit as described above wherein the stent is placed in a bronchial duct.

Another embodiment of the invention is a stent, use or kit as described above, wherein the stent is placed in a digestive duct such as esophagus, bowel, and/or biliary tract.

Another embodiment of the invention is a stent, use or kit as described above, wherein the stent is placed in a gynecological duct such as uterus, cervix, and/or vagina.

Another embodiment of the invention is a stent, use or kit as described above, wherein the stent is placed in a resection cavity or scar.

Another embodiment of the invention is a stent, use or kit as described above, wherein said rhodamine is a derivative of rhodamine.

Another embodiment of the invention is a medical stent at least partly covered with an expandable membrane over the expandable region, said membrane provided with a composition comprising rhodamine 123.

Another embodiment of the invention is a medical stent as described above wherein said membrane is biodegradable or non biodegradable material.

Another embodiment of the invention is a medical stent as described above wherein said membrane is at least partly formed from any of aliphatic polyester copolymers, elastomeric copolymers of epsilon -caprolactone and glycolide, elastomeric copolymers of E-caprolactone and lactide, copolymers of E-caprolactone and lactic acid, elastomeric copolymers of p-dioxanone and lactide, elastomeric copolymers of p-dioxanone and lactic acid, elastomeric copolymers of epsilon-caprolactone and p-dioxanone, elastomeric copolymers of p-dioxanone and trimethylene carbonate, elastomeric copolymers of trimethylene carbonate and glycolide, elastomeric copolymer of trimethylene carbonate and lactide, or elastomeric copolymer of trimethylene carbonate and lactic acid.

Another embodiment of the invention is a medical stent as described above, wherein said stent is at least partly made from a material which is biodegradable *in situ* and is impregnated with said composition.

Another embodiment of the invention is a medical stent as described above, wherein said stent comprises a magnesium based alloy.

Another embodiment of the invention is a medical stent as described above, wherein said stent is at least partly made from a material which is non-biodegradable.

Another embodiment of the invention is a medical stent as described above, wherein said membrane is at least partly provided with said composition.

Another embodiment of the invention is a use of a composition comprising rhodamine 123, for the preparation of a composition for providing a medical stent at least partly covered with an expandable membrane for inhibiting cell proliferation.

Another embodiment of the invention is a use of a composition comprising rhodamine 123 for the preparation of a composition for providing a medical stent at least partly covered with an expandable membrane for sensitizing proliferating cells to radiotherapy.

Another embodiment of the invention is a use of a composition comprising rhodamine 123 for the preparation of a composition for providing a medical stent at least partly covered with an expandable membrane for sensitizing a proliferating cells to chemotherapy.

Another embodiment of the invention is a kit comprising a) at least one medical stent at least partly covered with an expandable membrane and b) a composition comprising rhodamine 123.

Another embodiment of the invention is a medical stent, use or kit as described above, wherein said composition further comprises one or more slow release agents to facilitate slow release of inhibitor.

Another embodiment of the invention is a medical stent, use or kit as described above, wherein said slow release agent is any as defined above.

Another embodiment of the invention is a medical stent, use or kit as described above, wherein said rhodamine 123 is encapsulated a micro-capsule or nano-capsule.

Another embodiment of the invention is a medical stent, use or kit as described above wherein a nano-capsule is a nano-capsule as defined above.

Another embodiment of the invention is a medical stent as described above wherein a micro-capsule is a micro-capsule as defined above.

Another embodiment of the invention is a medical stent, use or kit as described above, wherein said rhodamine 123 is coupled to solubilising agent.

Another embodiment of the invention is a medical stent, use as described above, wherein said solubilising agent is a solubilizing agent as defined above.

Another embodiment of the invention is a medical stent, use or kit as described above, wherein said composition further comprises one or more polymers to facilitate attachment of the composition to the membrane and/or slow release of the composition.

Another embodiment of the invention is a medical stent, use or kit as described above, wherein said polymer is one or more of the polymers as defined above.

Another embodiment of the invention is a medical stent or a kit as described above, suitable for use in inhibiting cell proliferation.

Another embodiment of the invention is a medical stent or kit or a use as described above, wherein said cell proliferation is restenosis or stenosis.

Another embodiment of the invention is a medical stent, a kit or a use as described above, wherein said cell proliferation is cancer.

Another embodiment of the invention is a medical stent, a kit or a use as described above wherein said stent is placed in an artery or vein.

Another embodiment of the invention is a medical stent, a kit or a use as described above wherein said stent is placed in a bronchial duct.

Another embodiment of the invention is a medical stent, a kit or a use as described above, wherein said stent is placed in a digestive duct such as esophagus, bowel, and/or biliary tract.

Another embodiment of the invention is a medical stent, a kit or a use as described above, wherein said stent is placed in a gynecological duct such as uterus, cervix, and/or vagina.

Another embodiment of the invention is a medical stent, a kit or a use as described above, wherein said stent is placed in a malignant or benign resection cavity or scar.

Another embodiment of the invention is a stent, use or kit as described above, wherein said rhodamine is a derivative of rhodamine.

### DETAILED DESCRIPTION OF THE INVENTION

Unless defined otherwise, all technical and scientific terms used herein have the same meaning as is commonly understood by one of skill in the art.

The articles "a" and "an" are used herein to refer to one or to more than one, *i*.*e*. to at least one of the grammatical object of the article. By way of example, "a stent" means one stent or more than one stent

Throughout this application, the term "about" is used to indicate that a value includes the standard deviation of error for the device or method being employed to determine the value.

The recitation of numerical ranges by endpoints includes all integer numbers and, where appropriate, fractions subsumed within that range (*e*.*g*. 1 to 5 can include 1, 2, 3, 4 when referring to, for example, a number of stents, and can also include 1.5, 2, 2.75 and 3.80, when referring to, for example, doses).

The present invention relates to a stent provided with a composition comprising at least one type of inhibitor of ATP synthesis optionally together with at least one inhibitor of the pentose phosphate pathway (PPP) for use in treating stenosis and preventing restenosis in vascular ducts, and for use in treating cancerous tumours present in ducts such as the vascular ducts, bronchial ducts, biliary ducts, the oesophasgus, digestive tract, urethral duct, and uretheral duct etc, and for use in treating any disorder arising from the proliferation of cells in ducts. The stent may also be used for the treatment of cellular proliferation (*e*.*g*. cancer, stenosis and restenosis) in resectioned ducts *e*.*g*. oespohagus after surgical removal of cancer. The stent may also be used for the treatment of cellular proliferation (*e*.*g*. cancer, stenosis and restenosis) in resectioned cavities *e*.*g*. in the breast after removal of cancer, or in a post-operative scar. The composition can be a pharmaceutical composition. Where a particular use of a composition of the present invention is described, said use may be understood as a method.

The composition can be used for the treatment of proliferating cellular masses. The treatment may shrink the mass or may completely eradicate it. The treatment of proliferating cells to tissues may also be applied to regions which from which a proliferating mass has been surgically removed to reduce the possibility of relapse or regrowth.

The present invention also relates to a method for sensitising proliferating cells present in a cavity of a subject to treatment by radiotherapy and/or chemotherapy, comprising administering into the proliferating cell mass a composition comprising one or more inhibitors of ATP synthesis prior to said radiotherapy and/or chemotherapy.
The inventors have found that inhibitors of ATP synthesis and the PPP are avidly taken up by cells that rapidly profilerate. This property enables an inhibitor of cells to be employed proximal to the site of cell profileration, and to be selectively taken up by said proliferating cells in doses higher than by non-proliferating cells. This allows high doses of inhibitors, or extremely potent inhibitors to accumulate inside proliferating cells, while reducing or eliminating cell death of non-proliferating cells. This possibly allows for a reduction in the amount of active substance necessary on / within the stent to inhibit cell proliferation.

The inventors have also found that cell death in profilerating cell masses can be efficiently effected by administering inhibitors of ATP synthesis and optionally the PPP. The inventors have also found that some inhibitors which block aerobic, anaerobic or both anaerobic and aerobic ATP syntheses are both rapidly and selectively taken up, and are effective at killing rapidly proliferating cells, when part of a cell mass. The inventors have further found that administering inhibitors of ATP synthesis together with some inhibitors of the PPP to a proliferating cell mass leads to a still further effective cell death. An aspect of the invention is a stent that provides at least one inhibitor of ATP synthesis optionally together with at least one inhibitor of the PPP to a mass of cells. One embodiment of the present invention is a use of one or more inhibitors of ATP synthesis for the preparation of a composition for coating a stent, for the treatment of profilerating cells.

The stent allows treatment of cellular proliferation over a prolonged period. There is no requirement for the patient to be hospitalised during treatment, unlike conventional chemotherapy. It also allows treatment while the patient is fasting, (*e*.*g*. every night) and there is no competition towards ATP inhibition from the degradation products of ingested meals.

The term "rapidly profilerating cell" or "proliferating cell" as used herein refers to any type of cell that undergoes rapid cell division such as, for example, cancer cells, smooth muscle cells, stenosing cells, restenosing cells, and any rapidly proliferating cell. A collection of such cells form a cell mass.

The term "duct" as used herein refers to any walled cavity of a subject suitable for placing a medical stent therein. Such a duct may be narrowed by a medical condition such as stenosis, cancer, benign tumours, atherosclerosis, or invasion of a cancer originating from the wall or passing through the wall of a duct. Examples of ducts include, but are not limited to arteries, veins, bronchial ducts, biliary ducts, oesophasgus, digestive tract, urethral duct, and uretheral duct. The stent may also be placed inside tumour resection cavities such as breast and prostrate tissues after surgical removal of a proliferating cells. The stent may also be placed over a scar present in a cavity or duct, after removal of a cellular proliferation.

A "subject" according to the present invention may be any living body susceptible to treatment by a stent. Examples include, but are not limited to humans, dogs, cats, horses, cows, sheep, rabbits, and goats.

Where a stent is provided with a composition, it means the composition is deposited on, within the stent or on a membrane covering the stent, so the composition can released when the stent contacts the duct or resectioned cavity. The stent may be coated with the composition, Alternatively, the stent may be impregnated with composition, Alternatively, the stent may comprise cavities in which the composition resides. Alternatively, the stent may be at least partly covered with a membrane on which the composition is disposed. Various embodiments of the stent are described below.

A composition as used herein may comprise at least one type of inhibitor of ATP synthesis and optionally at least one inhibitor of the pentose phosphate pathway (PPP). The types of inhibitor and the pathway they inhibit is described in more detail below. According to the invention, a stent is provided with an inhibitor of oxidative phosphorylation which is rhodamine. In another preferred mode of the invention, a stent is provided with an inhibitor of glycolysis which is 2FDG. In another preferred mode of the invention, a stent is provided with an inhibitor of the TCA cycle which is fluoroacetate. In another preferred mode, the stent is provided with a combination of two or more of the aforementioned inhibitors; preferably the stent is provided with rhodamine and 2FDG. In a preferred mode, the stent is used to treat tumourous cancer. The therapy may be carried out in combination with low dose radiotherapy and/or chemotherapy.

A composition of the invention may comprise additional substances, such as, for example, those that facilitate the attachment of the inhibitor to the stent, those that release the inhibitor in a controlled manner *in situ*, and those that facilitate the functioning of the stent *in situ.* Such additional substances are known to the skilled artisan.

### Stents

Stents according to the invention may be any stent that is capable of being provided with a composition according to the invention. Stents have been extensively described in the art. For example they may be cylinders which are perforated with passages that are slots, ovoid, circular, regular, irregular or the like shape. They may also be composed of helically wound or serpentine wire structures in which the spaces between the wires form the passages. Stents may also be flat perforated structures that are subsequently rolled to form tubular structures or cylindrical structures that are woven, wrapped, drilled, etched or cut to form passages. A stent may also be combined with a graft to form a composite medical device, often referred to as a stent graft. A stent should capable of being coated with a composition described herein.

Stents may be made of biocompatible materials including biostable and bioabsorbable materials. Suitable biocompatible metals include, but are not limited to, stainless steel, tantalum, titanium alloys (including nitinol), and cobalt alloys (including cobalt-chromium-nickel alloys). Stents may be made of biocompatible and bioabsorbable materials such as magnesium based alloys. Bioabsorbable stents may inserted at the site of treatment, and left in place. The structure of the stent does not become incorporated into the wall of the duct being treated, but is degraded with time. Where the stent is made from biostable (non-absorbable) materials, the stent may be inserted for the duration of treatment and later removed.

Suitable nonmetallic biocompatible materials include, but are not limited to, polyamides, polyolefins (*i*.*e*. polypropylene, polyethylene etc.), nonabsorbable polyesters (i.e. polyethylene terephthalate), and bioabsorbable aliphatic polyesters (i.e. homopolymers and copolymers of lactic acid, glycolic acid; lactide, glycolide, para-dioxanone, trimethylene carbonate, epsilon -caprolactone, etc. and blends thereof), lactide capronolactone, poly(L-lactide) (PLLA), poly(D,L-lactide) (PLA), polyglycolide (PGA), poly(L-lactide-co-D,L-lactide) (PLLA/PLA), poly(L-lactide-co-glycolide) (PLLA/PGA), poly(D, L-lactide-co-glycolide) (PLA/PGA), poly(glycolide-co-trimethylene carbonate) (PGA/PTMC), polyethylene oxide (PEO), polydioxanone (PDS), polycaprolactone (PCL), polyhydroxylbutyrate (PHBT), poly(phosphazene), polyD,L-lactide-co-caprolactone) (PLA/PCL), poly(glycolide-co-caprolactone) (PGA/PCL), polyanhydrides (PAN), poly(ortho esters), poly(phoshate ester), poly(amino acid), poly(hydroxy butyrate), polyacrylate, polyacrylamid, poly(hydroxyethyl methacrylate), elastin polypeptide co-polymer, polyurethane, starch, polysiloxane and their copolymers.

Stents according to the present invention can be of any type known in the art suitable for delivery ATP inhibitors. As such these stents can be balloon expandable, self-expanding, provided with cavities etched into the framework of the stent for containing substances, stents provided with means for containing substances, bioabsorbable stents. The stent may also be made from different sorts of wires, for instance from polymeric biodegradable wires containing the active compound, interweaved with the metallic struts of the stent (balloon expendable or self-expandable stent).

Self expanding stents may be braided, from flexible metal, such as special alloys, from nitenol, from phynox. Self-expandable stents made from nitenol may be laser cut. One or more of the filaments that compose the self-expandable stent can be made from a polymer or a tube that elutes the anti-energetic compound.

Variations of stent and polymers are described in more detailed below.

Examples of stents include, but are not limited to, those described in US 4,733,665, US 4,800,882, US 4,886,062, US 5,514,154, US 6,398,806, EP 1 140 242, US 6,248,129, EP 1 217 969, EP 1 359 868, EP 1 349 517, EP 1 347 717, EP 1 318 765, EP 1 296 615, EP 1 229 864, EP 1 194 081, EP 1 191 904, EP 1 139 914, EP 1 087 701, EP 1 079 768, EP 1 018 985, EP 0 749 729, EP 0 556 850, EP 1 328 212, EP 1 322 256, EP 0 740 558, EP 1 251 800, EP 1 251 799, EP 1 235 856, EP 1 227 772, EP 1 123 065, EP 1 112 040, EP 1 094 764, EP 1 076 534, EP 1 065 993, EP 1 059 896, EP 1 059 894, EP 1 049 421, EP 1 027 012, EP 1 001 718, EP 0 986 416, EP 0 859 644, EP 0 740 558, EP 0 664 689, EP 0 556 850, EP 1 372 535, US 6,669,723, US 6,663,660, EP 1 065 996, US 6,652,577, US 6,652,575, EP 1 360 943, US 6,620,202, US 6,610,087, US 6,602,283, US 6,592,617, US 6, 585,758, US 6,585,753, EP 1 318 771, EP 1 318 768, US 6,579,308, US 2003/0109931, EP 1 163 889, EP 0 790 811, US 6,551,351, US 6,540,777, US 6,533,810, US 6,530,950, US 6,527,802, US 6,524,334, US 6,506,211, US 6,488,703, US 6,485,590, US 6,478,816, US 6,478,815, US 6,475,233, EP 1 251 891, US 6,471,720, US 6,428,569, EP 1 223 873, EP 0 758 216, US 6,416,543, US 6,641,538, EP 1 217 101, US 6,409,754, US 6,409,753, US 2002/0077592, EP 0 754 017, US 6,398,807, EP 1 207 815, EP 0 775 471, US 6,395,020, IS 6,391,052, US 6,387,122, US 6,379,379, US 6,355,070, US 6,348,065, EP 1 173 110, US 6,334,870, EP 1 163 889, IS 6,325,822, US 6,319,277, EP 1 144 042, EP 0 622 059, US 6,261,319, EP 1 112 039, US 6,251,134, US 6,240,978, US 6,217,607, US 6,193,744, US 6,174,328, EP 1 065 996, US 6,168,621, US 6,168,619, US 6,159,238, US 6,159,237, US 6,146,416, US 6,143,002, EP 0 986 416, EP 1 032 329, EP 1 019 107, EP 1 011 529, EP 1 011 528, US 6,071,308, EP 0 859 644, US 6,059,810, US 6,042,597, US 6,033,433, EP 0 979 059, US 6,022,371, US 5,993,483, US 6,957,974, US 5,594,744, WO 99/44535, EP 0 934 034, EP 0 934 033, US 5,922,019, WO 99/16388, EP 0 858 298, US 5,891,191, US 5,888,201, US 5,876,448, US 5,876,445, US 5,868,781, US 5,855,600, WO 98/55174, EP 0 746 375, US 5,824,045, US 5,800,511, EP 0 836 839, EP 0 767 685, US 5,683,488, EP 1 212 987, EP 1 212 987, EP 1 151 730, EP 1 151 730, EP 0 722 701, EP 1 236 447, EP 1 293 178, EP 1 236 449, EP 1 190 685, EP 1 138 280, EP 1 346 706, EP 1 330 993, EP 1 258 231, EP 1 325 717, EP 1 302 179, EP 1 095 634, EP 1 302 179, EP 1 295 615, EP 1 293 178, EP 1 266 638, EP 1 266 638, EP 1 260 197, EP 1 236 448, EP 1 236 446, EP 1 236 445, EP 1 258 231, EP 1 236 449, EP 1 236 448, EP 1 236 447, EP 1 236 446, EP 1 236 445, EP 1 112 724, EP 1 190 685, EP 1 179 323, EP 1 138 280, EP 1 112 724, EP 1 031 330, EP 0 937 442, EP 1 042 997, EP 1 031 330, EP 1 025 812, EP 1 000 590, EP 0 950 386, EP 0 873 734, EP 0 937 442, EP 0 864 302, EP 0 928 606, EP 0 864 302, EP 0 806 191, EP 1 212 988, EP 1 266 640, EP 1 266 639, EP 0 879 027, EP 1 226 798, US 6,656,215, EP 1 362 564, EP 0 904 009, EP 1 212 990, EP 1 155 664, EP 1 121 911, EP 1 266 640, EP 1 266 639, EP 1 031 329, EP 1 212 990, EP 1 212 988, EP 0 698 380, EP 1 155 664, EP 1 121 911, US 6,270,521, EP 1 031 329, EP 0 928 605, EP 0 928 605, EP 0 904 009, EP 0 903 123, EP 0 879 027, US 5,840,009, EP 0 839 506, US 5,741,324, EP 0 823 245, EP 0 698 380, EP 0 606 165, US 6,626,938, US 6,613,075, US 6.565,600, US 6,562,067, US 6,547,817, US 6,540,775, US 6,520,985, US 6,494,908, US 6,482,227, US 6,423,091, US 6,342,067, US 6,325,825, US 6,315,708, US 6,267,783, US 6,267,777, US 6,264,687, US 6,258,116, US 6,238,409, US 6,214,036, US 6,190,406, US 6,176,872, US 6,162,243, US 6,129,755, US 6,053,873, EP 0 904 009, US 6,019,778, EP 0 974 315, US 6,017,363, EP 0 951 877, EP 0 970 711, EP 0 785 807, US 6,013,019, EP 0 947 180, US 5,997,570, US 5,980,553, EP 0 951 877, US 5,938,682, US 5,895,406, US 5,891,108, US 5,882,335, US 5,792,172, US 5,782,906, EP 0 810 845, US 5,695,516, US 5,674,241, US 5,609,605, US 5,607,442, US 5,599,291, US 5,135,536, US 5,116,365, EP 0 378 151, US 4,994,071, EP 0 378 151, US 4,856,516.

### Polymers

It is an aspect of the invention that the stent is provided with at least one type of inhibitor of ATP synthesis and optionally at least one inhibitor of the PPP by way of at least partially coating the stent with a composition comprising a polymer. A polymer according to the present invention is any that facilitates attachment of the inhibitor(s) to the stent (*i*.*e*. stent and/or membrane) and/or facilitates the controlled release of said inhibitors.

Polymers suitable for use in the present invention are any that are capable of attaching to the stent and releasing inhibitor. They must be biocompatible to minimize irritation to the duct wall. Polymers may be, for example, film-forming polymers that are absorbable or non-absorbable. The polymer may be biostable or bioabsorbable depending on the desired rate of release or the desired degree of polymer stability.

Suitable bioabsorbable polymers that could be used include polymers selected from the group consisting of aliphatic polyesters, poly(amino acids), copoly(ether-esters), polyalkylenes oxalates, polyamides, poly(iminocarbonates), polyanhydrides, polyorthoesters, polyoxaesters, polyamidoesters, polylactic acid (PLA), polyethylene oxide (PEO), polycaprolactone (PCL), polyhydroxybutyrate valerates, polyoxaesters containing amido groups, poly(anhydrides), polyphosphazenes, silicones, hydrogels, biomolecules and blends thereof.

For the purpose of the present invention, aliphatic polyesters include homopolymers and copolymers of lactide (which includes lactic acid D-, L- and meso lactide), epsilon caprolactone, glycolide (including glycolic acid), hydroxybutyrate, hydroxyvalerate, para-dioxanone, trimethylene carbonate (and its alkyl derivatives), 1,4-dioxepan-2-one, 1,5-dioxepan-2-one, 6,6-dimethyl-1,4-dioxan-2-one and polymer blends thereof. Poly(iminocarbonate) for the purpose of this invention include as described by Kemnitzer and Kohn, in the Handbook of Biodegradable Polymers, edited by Domb, Kost and Wisemen, Hardwood Academic Press, 1997, pages 251-272. Copoly(ether-esters) for the purpose of this invention include those copolyester-ethers described in Journal of Biomaterials Research, Vol. 22, pages 993-1009, 1988 by Cohn and Younes and Cohn, Polymer Preprints (ACS Division of Polymer Chemistry) Vol. 30(1), page 498, 1989 (e.g. PEO/PLA). Polyalkylene oxalates for the purpose of this invention include Patent Nos. 4,208,511; 4,1-41,087; 4,130,639; 4,140,678; 4,105,034; and 4,205,399.

Polyphosphazenes, co-, ter- and higher order mixed monomer based polymers made from L-lactide, D,L-lactide, lactic acid, glycolide, glycolic acid, para-dioxanone, trimethylene carbonate and epsilon -caprolactone such as are described by Allcock in The Encyclopedia of Polymer Science, Vol. 13, pages 31-41, Wiley Intersciences, John Wiley & Sons, 1988 and by Vandorpe, Schacht, Dejardin and Lemmouchi in the Handbook of Biodegradable Polymers, edited by Domb, Kost and Wisemen, Hardwood Academic Press, 1997, pages 161-182.

Polyanhydrides from diacids of the form HOOC-C₆H₄-O-(CH₂)m-O-C₆H₄-COOH wherein m is an integer in the range of from 1 to 11, 3 to 9, 3 to 7, 2 to 6 or preferably 2 to 8, and copolymers thereof with aliphatic alpha-omega diacids of up to 8, 9, 10, 11 or preferably 12 carbons. Polyoxaesters polyoxaamides and polyoxaesters containing amines and/or amido groups are described in one or more of the following U.S. Patent Nos. 5,464,929; 5,595,751; 5,597,579; 5,607,687; 5,618,552; 5,620,698; 5,645,850; 5,648,088; 5,698,213 and 5,700,583; (which are incorporated herein by reference). Polyorthoesters such as those described by Heller in Handbook of Biodegradable Polymers, edited by Domb, Kost and Wisemen, Hardwood Academic Press, 1997, pages 99-118.

Other polymeric biomolecules for the purpose of this invention include naturally occurring materials that may be enzymatically degraded in the human body or are hydrolytically unstable in the human body such as fibrin, fibrinogen, collagen, gelatin, glycosaminoglycans, elastin, and absorbable biocompatible polysaccharides such as chitosan, starch, fatty acids (and esters thereof), glucoso-glycans and hyaluronic acid.

Suitable biostable polymers with relatively low chronic tissue response, such as polyurethanes, silicones, poly(meth)acrylates, polyesters, polyalkyl oxides (polyethylene oxide), polyvinyl alcohols, polyethylene glycols and polyvinyl pyrrolidone, as well as, hydrogels such as those formed from crosslinked polyvinyl pyrrolidinone and polyesters could also be used. Other polymers could also be used if they can be dissolved, cured or polymerized on the stent. These include polyolefins, polyisobutylene and ethylene-alphaolefin copolymers; acrylic polymers (including methacrylate) and copolymers, vinyl halide polymers and copolymers, such as polyvinyl chloride; polyvinyl ethers, such as polyvinyl methyl ether; polyvinylidene halides such as polyvinylidene fluoride and polyvinylidene chloride; polyacrylonitrile, polyvinyl ketones; polyvinyl aromatics such as polystyrene; polyvinyl esters such as polyvinyl acetate; copolymers of vinyl monomers with each other and olefins, such as etheylene- methyl methacrylate copolymers, acrylonitrile-styrene copolymers, ABS resins and ethylene-vinyl acetate copolymers; polyamides,such as Nylon 66 and polycaprolactam; alkyd resins; polycarbonates; polyoxymethylenes; polyimides; polyethers; epoxy resins, polyurethanes; rayon; rayon-triacetate, cellulose, cellulose acetate, cellulose acetate butyrate; cellophane; cellulose nitrate; cellulose propionate; cellulose ethers (i.e. carboxymethyl cellulose and hydoxyalkyl celluloses); and combinations thereof. Polyamides for the purpose of this application would also include polyamides of the form-NH-(CH₂)n-CO- and NH-(CH₂)x-NH-CO-(CH₂)y-CO, wherein
n is an integer in from 5 to 15, 7 to 11, 8 to 10 or preferably 6 to 13;
x is an integer in the range of from 5 to 14, 7 to 11, 8 to 10 or preferably 6 to 12; and
y is an integer in the range of from 3 to 18, 5 to 14, 6 to 10 or preferably 4 to 16. The list provided above is illustrative but not limiting.

Other polymers suitable for use in the present invention are bioabsorbable elastomers, more preferably aliphatic polyester elastomers. In the proper proportions aliphatic polyester copolymers are elastomers. Elastomers present the advantage that they tend to adhere well to the metal stents and can withstand significant deformation without cracking. The high elongation and good adhesion provide superior performance to other polymer coatings when the coated stent is expanded. Examples of suitable bioabsorbable elastomers are described in U.S. Patent No. 5,468,253. Preferably the bioabsorbable biocompatible elastomers based on aliphatic polyester, including but not limited to those selected from the group consisting of elastomeric copolymers of epsilon -caprolactone and glycolide (preferably having a mole ratio of epsilon -caprolactone to glycolide of from about 35:65 to about 65:35, more preferably 45:55 to 35:65) elastomeric copolymers of E-caprolactone and lactide, including L-lactide, D-lactide blends thereof or lactic acid copolymers (preferably having a mole ratio of epsilon -caprolactone to lactide of from about 35:65 to about 90:10 and more preferably from about 35:65 to about 65:35 and most preferably from about 45:55 to 30:70 or from about 90:10 to about 80:20) elastomeric copolymers of p-dioxanone (1,4-dioxan-2-one) and lactide including L-lactide, D-lactide and lactic acid (preferably having a mole ratio of p-dioxanone to lactide of from about 30:70 to about 70:30, 45:55 to about 55:45, and preferably from about 40:60 to about 60:40) elastomeric copolymers of epsilon - caprolactone and p-dioxanone (preferably having a mole ratio of epsilon -caprolactone to p-dioxanone of from about 40:60 to about 60:40 and preferably from about 30:70 to about 70:30) elastomeric copolymers of p-dioxanone and trimethylene carbonate (preferably having a mole ratio of p-dioxanone to trimethylene carbonate of from about 40:60 to about 60:40, and preferably from about 30:70 to about 70:30), elastomeric copolymers of trimethylene carbonate and glycolide (preferably having a mole ratio of trimethylene carbonate to glycolide of from about 40:60 to about 60:40 and preferably from about 30:70 to about 70:30), elastomeric copolymer of trimethylene carbonate and lactide including L-lactide, D-lactide, blends thereof or lactic acid copolymers (preferably having a mole ratio of trimethylene carbonate to lactide of from about 30:70 to about 70:30) and blends thereof. As is well known in the art these aliphatic polyester copolymers have different hydrolysis rates, therefore, the choice of elastomer may in part be based on the requirements for the coatings adsorption. For example epsilon -caprolactone-co-glycolide copolymer (45:55 mole percent, respectively) films lose 90% of their initial strength after 2 weeks in simulated physiological buffer whereas the epsilon -caprolactone-co-lactide copolymers (40:60 mole percent, respectively) loses all of its strength between 12 and 16 weeks in the same buffer. Mixtures of the fast hydrolyzing and slow hydrolyzing polymers can be used to adjust the time of strength retention.

The amount of coating may range from about 0.5 to about 20 as a percent of the total weight of the stent after coating and preferably will range from about 1 to about 15 percent. The polymer coatings may be applied in one or more coating steps depending on the amount of polymer to be applied. Different polymers may also be used for different layers in the stent coating. In fact it may be an option to use a dilute first coating solution as primer to promote adhesion of a subsequent coating layers that may contain inhibitor.

Additionally, a top coating can be applied to further delay release of the inhibitory pharmaceutical agent, or they could be used as the matrix for the delivery of a different pharmaceutically active material. The amount of top coatings on the stent may vary, but will generally be less than about 2000 micrograms, preferably the amount of top coating will be in the range of about micrograms to about 1700 micrograms and most preferably in the range of from about 300 micrograms to 1000 about micrograms. Layering of coating of fast and slow hydrolyzing copolymers can be used to stage release of the drug or to control release of different agents placed in different layers. Polymer blends may also be used to control the release rate of different agents or to provide desirable balance of coating (i.e. elasticity, toughness etc.) and drug delivery characteristics (release profile). Polymers with different solubilities in solvents can be used to build up different polymer layers that may be used to deliver different drugs or control the release profile of a drug. For example since epsilon -caprolactone-co-lactide elastomers are soluble in ethyl acetate and epsilon -caprolactone-co-glycolide elastomers are not soluble in ethyl acetate. A first layer of epsilon -caprolactone-co-glycolide elastomer containing a drug can be over coated with epsilon -caprolactone-co-glycolide elastomer using a coating solution made with ethyl acetate as the solvent. Additionally, different monomer ratios within a copolymer, polymer structure or molecular weights may result in different solubilities. For example, 45/55 epsilon -caprolactone-co-glycolide at room temperature is soluble in acetone whereas a similar molecular weight copolymer of 35/65 epsilon -caprolactone-co-glycolide is substantially insoluble within a 4 weight percent solution. The second coating (or multiple additional coatings) can be used as a top coating to delay the drug delivery of the drug contained in the first layer. Alternatively, the second layer could contain a different inhibitor to provide for sequential inhibitor delivery. Multiple layers of different inhibitors could be provided by alternating layers of first one polymer then the other. As will be readily appreciated by those skilled in the art numerous layering approaches can be used to provide the desired drug delivery.

The coatings can be applied by suitable methodology known to the skilled person, such as, for example, dip coating, spray coating, electrostatic coating, melting a powered form onto the stent. The coating may also be applied during the intervention by the interventional cardiologist on a bare stent. As some polymers (for instance polyorthoesters) need special conservation conditions (argon atmosphere and cold temperature), the drug with the coating may be delivered in a special packing. The MD would apply the coating on the bare stent surface -as it is sligthly sticky - just before introducing the premounted stent inside the patient duct or cavity.

Other examples of polymeric coatings, and coating methods are given in patent documents EP 1 107 707, WO 97/10011, US 6,656,156, EP 0 822 788, US 6,364,903, US 6,231,600, US 5,837,313, WO 96/32907, EP 0 832,655, US 6,653,426, US 6,569,195, EP 0 822 788 B1, WO 00/32238, US 6,258,121, EP 0 832,665, WO 01/37892, US 6,585,764, US 6,153,252.

### Non-polymeric coatings

Another aspect of the invention is a stent coated with a composition of the invention, wherein the presence of a polymer is optional. Such stents suited to polymeric and non-polymeric coatings and compositions are known in the art. These stents may, for example, have a rough surface, microscopic pits or be constructed from a porous material. Examples include, but are not limited to the disclosures of US 6,387,121, US 5,972,027, US 6,273,913 and US 6,099,561.

### Stent grafts

A stent may also be combined with a graft to form a composite medical device, often referred to as a stent graft. Such a composite medical device provides additional support for blood flow through weakened sections of a blood vessel. The graft element made be formed from any suitable material such as, for example, textiles such as nylon, Orlon, Dacron, or woven Teflon, and nontextiles such as expanded polytetrafluroethylene (ePTFE).

Stent grafts of the present invention may be coated with, or otherwise adapted to release the ATP inhibitor(s) (and optionally PPP inhibitor(s)) of the present invention. Stent grafts may be adapted to release such inhibitors by (a) directly affixing to the stent graft a composition according to the invention (*e*.*g*., by either spraying the stent graft with a polymer/inhibitor film, or by dipping the implant or device into a polymer/drug solution, or by other covalent or noncovalent means); (b) by coating the stent graft with a substance such as a hydrogel which will in turn absorb a composition according to the invention; (c) by interweaving a composition coated thread into the stent graft (*e*.*g*., a polymer which releases the inhibitor formed into a thread into the implant or device; (d) by inserting a sleeve or mesh which is comprised of or coated with a composition according to the present invention; (e) constructing the stent graft itself a composition according to the invention; or (f) otherwise impregnating the stent graft with a composition according to the invention.
The stent graft may be biodegradable, made from, but not limited to, magnesium alloy and starch.

Examples and methods of stent graft coating are provided in patent documents WO 00/40278, and WO 00/56247.

### Stent cavities

It is an aspect of the invention that the stent is provided with a composition of the invention which is present in a cavity formed in the stent. Stent in which cavities are present suitable for the delivery of biologically active material are known in the art, for example, from WO 02/060351 US 6,071,305, US 5,891,108.

### Biodegradable stents

Another aspect of the invention is a biodegradable (bioabsorbable) stent impregnated with a composition according to the present invention. The composition may be coated onto the stent or impregnated into the stent structure, said composition released *in situ* concomitant with the biodegradation of the stent. Suitable materials for the main body of the stent includes, but are not limited to poly(alpha-hydroxy acid) such as poly-L-lactide (PLLA), poly-D-lactide (PDLA), polyglycolide (PGA), polydioxanone, polycaprolactone, polygluconate, polylactic acid-polyethylene oxide copolymers, modified cellulose, collagen or other connective proteins or natural materials, poly(hydroxybutyrate), polyanhydride, polyphosphoester, poly(amino acids), hylauric acid, starch, chitosan, adhesive proteins, co-polymers of these materials as well as composites and combinations thereof and combinations of other biodegradable polymers. Biodegradable glass or bioactive glass is also a suitable biodegradable material for use in the present invention. A composition of the present invention may be incorporated into a biodegradable stent using known methods. Examples of biodegradable stents known in the art, include, but are not limited to the those disclosed in US 2002/0099434, US 6,387,124 B1, US 5,769,883, EP 0 894 505 A2, US 653,312, US 6,423,092, US 6,338,739 and US 6,245,103, EP 1 110 561. Biodegradable stents may also be made from a metal (lanthanide such as, but not limited to magnesium or magnesium alloy), or an association of organic and non-organic material (such as, but not limited to a magnesium based alloy combined with starch).

### Stent-membrane

Another aspect of the present invention is a stent provided with an expandable membrane, said membrane provided with a composition described herein. The expandable membrane may be provided over at least part of the expandable portion of the stent or over at least part of the struts. The membrane extends the contact of the composition with the wall of the treated duct or cavity (*e*.*g*. tubular structure such as esophagus, closed structure such as uterus cavity, or artificial cavity such as tumorectomy cavity). A problem with coating conventional stents can be the small size of the struts which are narrow to allow ease of expansion. Some treatments require doses of composition which are beyond the capacity of the stent struts. Increasing the strut size to accommodate larger doses compromises expandability and affects the weight and size of the collapsed stent. For example, the struts of a stent used to treat proliferating cells in a coronary vessel (*e*.*g*. 3 mm inner diameter) cover approximately 15 % of the vessel surface. Such stents may be suitable for the treatment of growths with a depth inferior to 1 mm. However, when one has to treat an esophageal tumour with a tumour thickness of 5 mm, 1 cm and 4-6 cm long for instance, one needs a certain amount of active substance which cannot be delivered by a stent where the active substance covers only the struts. There will be insufficient drug transfer in such a situation in order to obtain any minimal effect. A membrane which fits over the stent, which membrane is coated or filled with composition allows a greater capacity of inhibitor owing to the continuous area contacting the duct wall, and the possibility to impregnate or fill a lumen in the membrane with a significant quantity of composition.

The membrane can be made from any suitable exandable material. Examples of suitable expandable materials include aliphatic polyester elastomers. In the proper proportions aliphatic polyester copolymers are expandable. Examples of suitable bioabsorbable expandable polymers are described in U.S. Patent No. 5,468,253. Preferably the bioabsorbable biocompatible expandable polymers are based on aliphatic polyester, including but not limited to those selected from the group consisting of elastomeric copolymers of epsilon -caprolactone and glycolide (preferably having a mole ratio of epsilon -caprolactone to glycolide of from about 35:65 to about 65:35, more preferably 45:55 to 35:65) elastomeric copolymers of E-caprolactone and lactide, including L-lactide, D-lactide blends thereof or lactic acid copolymers (preferably having a mole ratio of epsilon -caprolactone to lactide of from about 35:65 to about 90:10 and more preferably from about 35:65 to about 65:35 and most preferably from about 45:55 to 30:70 or from about 90:10 to about 80:20) elastomeric copolymers of p-dioxanone (1,4-dioxan-2-one) and lactide including L-lactide, D-lactide and lactic acid (preferably having a mole ratio of p-dioxanone to lactide of from about 30:70 to about 70:30, 45:55 to about 55:45, and preferably from about 40:60 to about 60:40) elastomeric copolymers of epsilon - caprolactone and p-dioxanone (preferably having a mole ratio of epsilon -caprolactone to p-dioxanone of from about 40:60 to about 60:40 and preferably from about 30:70 to about 70:30) elastomeric copolymers of p-dioxanone and trimethylene carbonate (preferably having a mole ratio of p-dioxanone to trimethylene carbonate of from about 40:60 to about 60:40, and preferably from about 30:70 to about 70:30), elastomeric copolymers of trimethylene carbonate and glycolide (preferably having a mole ratio of trimethylene carbonate to glycolide of from about 40:60 to about 60:40 and preferably from about 30:70 to about 70:30), elastomeric copolymer of trimethylene carbonate and lactide including L-lactide, D-lactide, blends thereof or lactic acid copolymers (preferably having a mole ratio of trimethylene carbonate to lactide of from about 30:70 to about 70:30) and blends thereof. As is well known in the art these aliphatic polyester copolymers have different hydrolysis rates, therefore, the choice of expandable polymers may in part be based on the requirements for the membrane. For example epsilon -caprolactone-co-glycolide copolymer (45:55 mole percent, respectively) films lose 90% of their initial strength after 2 weeks in simulated physiological buffer whereas the epsilon -caprolactone-co-lactide copolymers (40:60 mole percent, respectively) loses all of its strength between 12 and 16 weeks in the same buffer. Mixtures of the fast hydrolyzing and slow hydrolyzing polymers can be used to adjust the time of strength retention.

An example of a stent-membrane is a self-expanding stent, coated with a polymeric membrane, suitable for placement inside the oesophagus in the vicinity of an esophageal tumour. The membrane may be coated with any of the above mentioned compositions. According to one aspect of the invention, the membrane is coated with a composition comprising FDG or oxamate and Rhodamine 123.

### Medical treatments

The inventors have found that an advantage of using compounds that inhibit ATP synthesis and compounds that inhibit the PPP when present, is that they are selectively absorbed by the rapidly proliferating cells.

As mentioned above, stenosis and prevention of restenosis conditions are susceptible to treatment by a stent according to the present invention.

According to the present invention, stents may also be placed on or adjacent to tumoral tissues for treatment of a tumour. Examples of tumours suitable for treatment according to the invention include biliary tract adenocarcinoma, esophageal epidermoid or adenocarcinoma, colon adenocarcinoma, bronchial epidermoid or adeno-carcinomas, etc.. Rapidly dividing tumour cells thus take up the inhibitory compounds according to the invention, so leading to cell death. In some cases, these anti-tumour stents are self-expanding because they ensure a mechanic maintenance of the patency of the lumen which is infiltrated by cancer. For example, a stent of the invention may be introduced at an early stage in the treatment of esophageal cancer, where only superficial lesions are present. The stent can be further removed and the lesion be followed-up by esophagoscopy.

The same type of therapy applies to biliary duct cancer, to bronchial cancer, where cancer invades the lumen. If a stent is placed, it will be invaded very rapidly by the cancer. Therefore, in the treatment of cancer, fully covered stents (stent grafts) may be used sometimes. Especially for cancer therapy, one could incorporate high concentrations of ATP inhibitors, within slow release polymers that will elute for months, for instance, in case of palliative therapy.

The stent may also be placed *in situ* after the removed of a tumour. For example, after surgical removal of an oesophageal cancer, a stent may be placed in the area of the esophageal suture to kill cells possibly remaining after surgery. Similarly, a stent may be placed in a resectioned cavity after removal of a tumour e.g. after removal of a breast cancer or a prostrate cancer.

Another aspect of the present invention is a stent according to the invention for the treatment of conditions relating to cell proliferation, wherein the subject is a diabetic. The inventors have found that the rate of uptake of inhibitors of ATP synthesis is possibly increased in diabetic subjects.

The present invention is useful for treating any animal in need including humans, livestock, domestic animals, wild animals, or any animal in need of treatment. Examples of an animal is human, horse, cat, dog, mice, rat, gerbil, bovine species, pig, fowl, camelidae species, goat, sheep, rabbit, hare, bird, elephant, monkey, chimpanzee etc. An animal may be a mammal.

### ATP synthesis inhibitors

The ATP synthesis inhibitors of the present of the invention may be any inhibitor that inhibits a pathway directly or indirectly leading to ATP synthesis, or derivatives thereof, or salts thereof. The composition of the present invention comprise an inhibitor of glycolysis or TCA cycle, or oxidative phosphorylation. The composition may comprise inhibitors towards any two or more of the above mentioned pathways. The composition may optionally comprise an inhibitor of the PPP.

### Glycolysis

An example of a pathway involved in anaerobic ATP synthesis is glycolysis. Enzymes associated with this pathway are known in the art and include hexokinase, glucokinase (in tumors or in rapidly proliferating tissues), phosphoglucose isomerase, phosphofructokinase, aldolase, triose phosphate isomerase, glyceraldehydes 3-phosphate dehydrogenase, phosphoglycerate kinase, phosphoglyceromutase, enolase, pyruvate kinase and, indirectly, lactate dehydrogenase (lactate metabolism). It is an aspect of the invention that an inhibitor of anaerobic ATP synthesis is an inhibitor of an enzyme associated with the glycolytic pathway. Inhibitors of the glycolytic pathway are any known in the art.

Inhibitors of hexokinase may be configurational isomers of monosaccharides modified at C-6 by substitution (replacement) or removal of 6-OH (the hydroxyl group). An example of a substituent is a blocking moiety - for example, an atom from the halogen family, such as fluorine (6-fluoro-D-glucose). Another example of a substituent is a thiol group. Monosaccharides modified at C-6 by removal of 6-OH will not be transformed by hexokinase or glucokinase (see below) to glucose-6-phosphate and can potentially block both enzymes.

Inhibitors of hexokinase are any known in the art and may include, but are not limited to any of the following:
- 6-fluoro-D-glucose, 6-bromo-D-glucose, 6-chloro-D-glucose, 6-O-methyl-D-glucose, 6-Thio-D-glucose, 6-deoxy-D-glucose, and any derivative known in the art.
- C-6 substituted derivatives of other hexose ring pyranoses (mannopyranoses, galactopyranoses). Examples include 6-deoxy-6-fluoro-D-mannose, and any known in the art.
- Various halogenated (fluoro, bromo, chloro-) C6 sugars derivatives such as gluconolactones, glucuronic acid, glucopyranoside, and their phosphate derivatives, and any known in the art. Halogenated glucosides may also be delivered indirectly to the cell, by compounds such as glucoronides with halogenated glycosides at the C-1 position (once in the cell, glucoronidases will cleave it, and deliver active hexose in the cell).
Preferably, an inhibitor of hexokinase is 6-deoxy-6-fluoro-D-glucose and its derivatives.

Inhibitors of glucokinase may be any in the art. They include, and are not limited to mannoheptulose , mannoheptose, glucoheptose, N-acetylglucosamine. Glucokinase is predominantly present in tumours only.

Inhibitors of phosphoglucoisomerase: Phosphoglucoseisomerase transforms glucose 6-phosphate to fructose 6-phosphate. Such transformation requires the presence of an hydroxyl group at C-2. Therefore, analogs without hydroxyl or having the hydroxyl properly blocked will not undergo isomerization by phosphoglucose isomerase.
Another way to inhibit isomerization by phosphoglucose isomerase is by modifying the glucose 6-phosphate at C-1 or C-5 by substituting hydroxyl with a halogenated atom (fluorine, glucosyl fluoride), or by simple deoxygenation to 1-deoxy-D-glucose.

Inhibitors of phosphoglucoisomerase are any known in the art and may include, but are not limited to any of the following:
C2 substituted D-hexoses, such as 2-deoxy-2-halogeno-D-hexoses, such as 2-deoxy-2-fluoro-D-glucose (2FDG), 2-chloro-2-deoxy-D-glucose, 2-bromo-D-glucose, 2-iodo-D-glucose, 2-deoxy-2,2-difluoro-D-arabino-hexose, 2-deoxy-2-fluoro-D-mannose, 2-deoxy-D-arabino-hexose, 2-Deoxy-2-fluoro-D-galactose, 1,6-anhydro-2-deoxy-2-fluoro-beta-D-glucopyranose (1-6-anhydrosugar), 2-amino-2-deoxy-D-glucose (glucose amine), 2-amino-2-deoxy D galactose (galactosamine), 2-amino-2-deoxy-D-mannose (mannosamine), 2-deoxy-2-fluoro-D-mannose, 2-deoxy-2-fluoro-D-galactose, 2-deoxy-D-arabino-hexose, 2-deoxy-2,2-difluoro-D-arabino-hexose, 2-deoxy-2-fluoro-D-glucose 1-Phosphate, 2-deoxy-2-fluoro-D-glucose 6-P, 2-deoxy-2-fluoro-D-glucose 1,6 biphosphate, 2-deoxy-2-fluoro-D-mannose 1-P, 2-deoxy-2-fluoro-D-mannose 6-P, 2-deoxy-2-fluoro-D-mannose 1,6-biphosphate, nucleotide diphosphate (for example uridine di-P)-2deoxy-2-fluoro-D-glucose, mannose.
C-2-halogen substituted, and NH3 substituted derivatives of D-Glucose 6-phosphate, 2-deoxy-2-fluoro-2-D-glucose-6-phosphate, 2-chloro-2-deoxy-D-glucose-6-phosphate, 2-deoxy-D-arabino-hexose-6-phosphate, D-glucosamine-6-phosphate, 2-deoxy-2-fluoro-2-D-manose-6-P, and any known derivatives.
C-2 halogenated derivatives of hexose ring pyranoses (mannopyranoses, galactopyranoses), for instance C-2-deoxy-2- fluoro-D-pyranoses, and any known in the art.
Halogenated (fiuoro, bromo, chloro, iodo) C2 sugars derivatives such as gluconolactones, glucuronic acid, glucopyranoside, and their phosphate derivatives.
Modification at C-1 or C-5: replacement of hydroxyl by fluorine or deoxygenation or replacement by a sulfur group in C-5, such as but not limited to glucosyl fluoride, 1-deoxy-D-glucose, 5-thio-D-glucose.
6-aminonicotinamide (6AN), indirectly by the inhibition of the PPP.

Inhibitors of phosphofructokinase (or fructose-6-P kinase) are any known in the art and may include, but are not limited to any of the following:
- Acidosis-inducing agents, 2-deoxy-2-fluoro-D-glucose, citrate and halogenated derivatives of citrate, fructose 2,6-bisphosphate, bromoacetylethanolamine phosphate analogues (N-(2-methoxyethyl)-bromoacetamide, N-(2-ethoxyethyl)-bromoacetamide, N-(3-methoxypropyl)-bromoacetamide).

Inhibitors of aldolase: Analogs blocking the aldolase cleavage, thus blocking formation of trioses from fructose 1,6-bisphosphate require the presence of hydroxyl groups at C-3 and C-4. Thus, for example, 3-deoxy or 3-fluoro-D-glucose or 4-deoxy or 4-fluoro-D-glucose can be transformed to 4-fluoro-D-fructose 1,6-bisphosphate, which will not be cleaved by aldolase but will block it. Inhibitors of glyceraldehyde 3P deshydrogenase are any known in the art and may include, but are not limited to any of the following:
Iodoacetate, pentalenolactone, arsenic, 1,1-difluoro-3-phosphate-glycerol.

Inhibitors of the transformation chain of glyceraldehyde (glyceraldehyde 3P deshydrogenase, phosphoglycerate kinase, phosphoglycerate mutase, enolase) are any which act at any step where phosphorylation is involved. Such inhibitors are any known in the art and may include, but are not limited to any of the following: either 2-fluoro (or iodo, or thio, or methoxy) or 3-fluoro (or 3,3 difluoro, 3-iodo, 3-carboxylo-, 3-thio)-glyceraldehydes or glycerates, 3-fluoro-2-phosphoglycerate, also, phosphothioesters or other phosphorous-modified analogs can block the transformations of glyceraldehyde.

Inhibitors of pyruvate kinase are any known in the art. Alternatively, a composition comprising serine or fructose 1,6-diP shifts the glycolytic pathway towards the TCA cycle; thus a composition of the invention comprises serine and an inhibitor of the TCA such as fluoroacetate or an inhibitor of the oxidative phosphorilation such as rhodamine.

Inhibitors of pyruvate carboxylase and PEP carboxylase, triose phosphate isomerase, phosphoglycerate kinase, enolase, phosphoglycerate mutase and triose phosphate isomerase are any known in the art.

Inhibitors of lactate deshydrogenase are any known in the art and may include, but are not limited to oxamate, 2-fluoro-propionic acid or it salts; 2,2-difluoro-propionic acid, pyruvate modified at C-3 such as, but not limited to 3-halo-pyruvate, 3-halopropionic acid and 2-thiomethylacetic acid.

Preferably, an inhibitor of glycolysis is any of 2FDG, oxamate and iodoacetate.

Glycolysis is the main pathway for anaerobic ATP synthesis. Tumours switch to anaerobic ATP synthesis by metabolizing the well-distributed glucose among others in order to provide nucleotides through the PPP pathway. It is known that proliferating masses which are partly under anaerobic type respiration are more resistant to radiation or chemotherapy. Therefore, by locally inhibiting the glycolysis pathway, anaerobic respiration which is the principal energy pathway of poorly oxygenated cells is inhibited, leading to increased cell death of hypoxic proliferating cells. The proliferation of non-hypoxic cells is slowed as well owing to the shutdown of this primary energy pathway.

### TCA cycle inhibitors

An example of a pathway involved in aerobic ATP synthesis is the tricarboxylic acid, TCA cycle (Krebs cycle). Enzymes associated with this pathway are known in the art and include pyruvate dehydrogenase complex, citrate synthase, aconitase, isocitrate lyase, alpha-ketoglutarate dehydrogenase complex, succinyl CoA synthetase, succinate dehydrogenase, fumarase, malate synthase, malate dehydrogenase, glutaminase and glutamate dehydrogenase (the later 2 indirectly). It is an aspect of the invention that an inhibitor of aerobic ATP synthesis is an inhibitor of an enzyme associated with the TCA cycle. Inhibitors of the TCA cycle are any known in the art.

General inhibitors of TCA: The availability of reduced and oxidized forms of nicotinamide adenine dinucleotide (NAD+ and NADH) is important for the TCA and depletors of NAD+ and NADH+ would be inhibitors of the TCA cycle. Depletors of NAD⁺ and/or NADH include Hypoglycin A and its metabolite methylenecyclopropylacetic acid, ketone bodies (D(-)-3-hydroxybutyrate), alloxan, PNU and any other substance known in the art.

Inhibitors of pyruvate dehydrogenase are any known in the art and may include, but are not limited to any of the following:
Halo-pyruvate (*e*.*g*. 3-fluoropyruvate, 3-chloropyruvate, 3-bromopyruvate, 3-iodopyruvate) Compounds of the general formula:

   X - CH₂ - CO- COOH

   wherein X represents a halide, a sulphonate, a carboxylate, an alkoxide, or an amine oxide.
   X may be a halide selected from the group consisting of fluoride, bromide, chloride and iodide.
   X may be a sulphonate selected from the group consisting of triflate, mesylate and tosylate. X may be an amine oxide that is dimethylamine oxide.

### Compounds of the general formula:

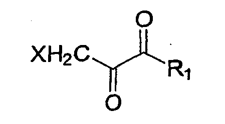

wherein X represents a halide, a sulphonate, a carboxylate, an alkoxide, or an amine oxide.
X may be a halide selected from the group consiting of fluoride, bromide, chloride and iodide.
X may be a sulphonate selected from the group consisting of triflate, mesylate and tosylate. X may be an amine oxide that is dimethylamine oxide.
R₁ may be OR, H, N(R")2, C1-C6 alkyl, C6-C12 aryl, C1-C6 heteroalkyl, or a C6-C12 heteroaryl. Independently, R" may represent H, C1-C6 alkyl, or C6-C12 aryl. Independently, R may be H, alkali metal, C1-C6 alkyl, C6-C12 aryl or C(O)R'; and R' may represent H, C1-C20 alkyl or C6-C12 aryl.

Other inhibitors are arsenite, dichlorovinyl-cysteine, p-benzoquinone, thiaminase and any others known in the art.

Inhibitors of citrate synthetase are any known in the art and may include, but are not limited to any of the following:
Fluoroacetate (an its derivative fluoroacetyl-CoA), any halogenated acetyl-CoA, fluoroacetamide, fluorocrotonate, halogenated ketone bodies (for instance, chloroacetoacetate, fluoroacetoacetate, fluorohydroxybutyrate, chlorohydroxybutyrate, bromohydroxybutyrate), halogenated acetone, halogenated acetic acid (for example chloracetic acid), halogenated oleate (an analogue of ketone bodies) and any known in the art.

Inhibitors of aconitase are any known in the art and may include, but are not limited to any of the following:
Fluorocitrate, fluorocitrate 2R, 3R, and any other halogenated citrate (bromocitrate, chlorocitrate).

Inhibitors of isocitrate dehydrogenase are any known in the art and may include, but are not limited to any of the following:
DCVC (dichlorovinyl-cysteine)

Inhibitors of succinate dehydrogenase are any known in the art and may include, but are not limited to malonate, DCVC, Pentachlorobutadienyl-cysteine (or PCBD-cys), 2-bromohydroquinone, 3-nitropropionic acid, cis-crotonalide fungicides.

Inhibitors of succinyl CoA synthetase, alpha ketoglutarate dehydrogenase complex, fumarate hydratase (fumarase), malate dehydrogenase are any known in the art.

Inhibitors of glutaminase are any known in the art and may include, but are not limited to 6-diazo-5-oxo-L-norleucine (DON).

Inhibitors of glutamate dehydrogenase are any known in the art.

Other inhibitors of the TCA cycle include glu-hydroxyoxamate, p-chloromercuriphenylsulphonic acid (impermeant thiol agent), L-glutamate gamma-hydroxamate, p-chloromercuriphenylsulphonic acid, acivicin (alpha-amino-3-chloro-4,5-dihydro-5-isoxazoleacetic acid), halogenated glutamine and glutamate.

Several other compounds may block the production of ATP at the level of the TCA cycle after compound transformation. Indeed, most amino-acids may be degraded to enter the TCA cycle at various places. Therefore, most of the aminoacids used in an halogenated formulation will be able to block the TCA cycle by being degraded to one of the TCA products (halogenated glutamate, glutamine, histidine, proline, arginine, valine, methionine, threonine, isoleucine, aspartate, tyrosine, phenylalanine, asparagine, aspartate, alanine, glycine, cysteine, serine, threonine). Some of the amino-acids will be transformed into ketone bodies. These amino-acids (leucine, lysine, phenylalanine, tyrosine) in an halogenated presentation will interact at the same sites where halogenated ketone bodies interact as described previously. Finally, some amino-acids in an halogenated formulation (tryptophan, leucine, isoleucine) will be directly transformed into acetyl-CoA and will block the TCA at the level of citrate synthase - aconitase.

Where more than one inhibitor of the TCA is present in a composition, preferably, one inhibitor is directed towards the upper half of the TCA cycle, which is characterised by providing no redox products such as NADH, NADPH, or FADH₂ (*e*.*g*. enzymes pyruvate dehydrogenase, citrate synthase, aconitase) and another inhibitor is directed towards the lower half of the TCA cycle, which is characterised by providing redox products such as NADH, NADPH, or FADH₂ (*e*.*g*. enzymes isocitrate lyase, alpha-ketoglutarate dehydrogenase complex, succinyl CoA synthetase, succinate dehydrogenase, malate synthase, glutaminase). Examples of a combination of inhibitor includes fluoroactetate and malonate.

### Fluorocitrate and derivatives

According to a preferred embodiment of the invention, a TCA cycle inhibitor of the invention has a formula (I): 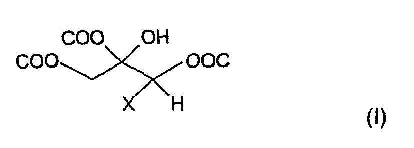 where X may be halide, a sulfonate, a carboxylate, an alkoxide, an amine oxide or a OH. The halide may be selected from the group consisting of: fluoride, bromide, chloride, and iodide. The sulfonate may be selected from the group consisting of: triflate, mesylate and tosylate. The carboxylate may be selected from the group consisting of: methoxylate and ethyloxylate. The alkoxide may be selected from the group consisting of: methoxide and ethoxide. The amine oxide is dimethylamine oxide. According to one aspect of the invention, the stereochemistry is 2R, 3R.

### Fluoroacetate and derivatives

TCA cycle inhibitors also includes substances which are converted into inhibitors of the TCA cycle such as, for example fluoroacetate and derivatives. According to a preferred embodiment of the invention, a TCA cycle inhibitor of the invention has a formula (II): 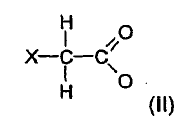 where X may be halide, a sulfonate, a carboxylate, an alkoxide, or an amine oxide, a OH. The halide may be selected from the group consisting of: fluoride, bromide, chloride, and iodide. The sulfonate may be selected from the group consisting of: triflate, mesylate and tosylate. The carboxylate may be selected from the group consisting of: methoxylate and ethyloxylate. The alkoxide may be selected from the group consisting of: methoxide and ethoxide. The amine oxide may be dimethylamine oxide.
Preferably, an inhibitor of the TCA cycle is any of fluoroacetate, fluorocitrate bromopruvate, arsenite, acetoacetate, and betahydroxy butyrate.

The requirement for both energy and building blocks such as lipids, anino acids, nucleic acids by rapidly proliferating cells means the TCA cycle is highly active. The inventors have found that the cells become susceptible to uptake of substrates of the TCA cycle such as acetate, much more so than non-proliferating cells. Therefore, treatment of readily proliferating cells with inhibitors of the TCA cycle benefits from rapid and selective inhibitor uptake, and cell death owing to the inhibition of the central anabolic pathway. More efficient cell death may be achieved in combination with other energy-producing pathways such as glycolysis and oxidative phosphorylation. Simultaneous inhibition of the PPP also shuts down an important anabolic pathway.

### Simultaneous inhibition of other pathways

According to an aspect of the invention a TCA cycle inhibitor is capable of inhibiting at least 3 cellular mechanisms of proliferating cells simultaneously. This may be achieved by blocking, for example, aconitase from the TCA cycle. The inventors have realised that the use of an aconitase inhibitor such as, for example, fluorocitrate (or fluoroacetate which is later converted into fluorocitrate) can inhibit other important pathways such as fatty acid synthesis at the level of ATP-citrate lyase and calcium intracellular signalling through derivatives accumulation.

### Oxidative phosphorylation

An example of a pathway involved in aerobic ATP synthesis is oxidative phosphorylation. Enzymes associated with this pathway are known in the art and include enzyme complex I (NADH coenzyme Q reductase), II (succinate-coenzyme Q reductase), III (coenzyme Q cytochrome C reductase), IV (cytochrome oxydase), and V (FO-F1, ATP synthase). It is an aspect of the invention that an inhibitor of aerobic ATP synthesis is an inhibitor of an enzyme associated with oxidative phosphorylation.

Inhibitors of enzyme complex I are any known in the art and may include, but are not limited to any of the following: tritylthioalanine, carminomycin, and piperazinedione, rotenone, amytal, 1-methyl-4-phenylpyridinium (MPP+), paraquat, methylene blue, Ferricyanide (the later 2 are electron acceptors).

Inhibitors of enzyme complex II are any known in the art.

Inhibitors of coenzyme Q are any known in the art.

Inhibitors of enzyme complex III are any known in the art and may include, but are not limited to myxothiazol, antimycin A, ubisemiquinone, cytochrome C, 4,6-diaminotriazine derivatives, metothrexate or electron acceptors such as phenazine methosulfate and 2,6-Dichlorophenol-indophenol.

Inhibitors of enzyme complex IV are any known in the art and may include, but are not limited to cyanide, hydrogen sulfide, azide, formate, phosphine, carbon monoxide and electon acceptor ferricyanide.

Inhibitors of enzyme complex V are any known in the art and may include, but are not limited to VM-26 (4'-demethyl-epipodophyllotoxin thenylidene glucoside), tritylthioalanine, carminomycin, piperazinedione, dinitrophenol, dinitrocresol, 2-hydroxy-3-alkyl-1,4-naphtoquinones, apoptolidin aglycone, oligomycin, ossamycin, cytovaricin, naphtoquinone derivatives (*e*.*g*. dichloroallyl-lawsone and lapachol), rhodamine, rhodamine 123, rhodamine 6G, carbonyl cyanide p-trifluoromethoxyphenylhydrazone, valinomycin, rothenone, safranine O, cyhexatin, DDT, chlordecone, arsenate, pentachlorophenol, benzonitrile, thiadiazole herbicides, salicylate, cationic amphilic drugs (amiodarone, perhexiline), gramicidin, calcimycin, pentachlorobutadienyl-cysteine (PCBD-cys), trifluorocarbonylcyanide phenylhydrazone (FCCP).

Where rhodamine, rhodamine 123, or rhodamine 6G are present in the composition, they may be used as inhibitors of oxidative phosphorylation, and not as a dye for homogeneous coating control, or for photodynamic therapy, for instance. Therefore, where rhodamine compounds are used, the treatment according to the invention is not in combination with rhodamine based imaging or light based treatment.

Other inhibitors of oxidative phorphorylation may include atractyloside, DDT, free fatty acids, lysophospholipids, n-ethylmaleimide, mersanyl, p-benzoquinone.

Preferably, an inhibitor of oxidative phosphorylation is any of rhodamine, dinitrophenol, and rotenone.

Inhibition of oxidative phosphorylation surprisingly leads to an inhibition of proliferation; proliferating cells do not obtain energy via other pathways and recover. The inventors have also found that inhibition of both oxidative phosphorylation and glycolysis is further effective against proliferating cells. This might be explained by the knockout of anaerobically synthesising cells. *In situ*, therefore, a combined treatment might be effective against a mass of proliferating cells comprising well oxygenated at the periphery, and cells towards the centre which are not well supplied with blood and respire anaerobically.

Because inhibitors of oxidative phorphorylation are extremely toxic to a subject in the doses needed for efficacy via the systemic route, such inhibitors have been largely overlooked for effective treatment of conditions such as cancer. For example, it is known that the LD10 (the dose which kills 10% of animals) for rhodamine 123 in rodents is 20 mg/kg. It is also known that the maximal tolerated dose of Rhodamine 123 delivered intravenously in man is 95 mg/m². For a 70 kg patient, the maximum dose of systemically delivered rhodamine is about 150 mg. Operating at the maximum, non-lethal dose, clinical trials have shown no useful effect on prostate tumours. There was an accumulation of rhodamine in the prostate of the patients, but it was not sufficient to provoke a significant response.

By locally delivering oxidative phosphorylation inhibitors on a stent, the delivery period is prolonged *i*.*e*. the inhibitors are not cleared by the liver, and the dose received by the tumour is effectively higher than using systemic delivery. Furthermore, the lethal dose can be greatly exceeded. For instance delivering 40 or 50 mg of rhodamine leads to tumour death, and not just to a slow down in tumour growth. Such a dose, delivered systemically, would require 100g of rhodamine, more than 1000 times the lethal dose. Thus, a stent coated with an oxidative phorphosrylation inhibitor increases the effective dose to the tumour and permits greater than lethal dosing.

Furthermore, the oxidative phosphorylation inhibitor coated stent may be used in combination with radiotherapy, where a lower dose of inhibitor may be used.

### Pentose phosphate pathway inhibitors

Enzymes associated with the pentose phosphate pathway are known in the art and include glucose-6-phosphate dehydrogenase, lactonase, 6-phosphogluconate dehydrogenase, phosphopentose isomerase, phosphopentose epimerase, transketolase, and transaldolase.

Inhibitors of glucose 6P dehydrogenase are any known in the art and may include, but are not limited to any of the following halogenated (fluorinated), D-hexoses (e.g. 2-Amino-2-deoxy-D-glucose-6-phophate (D-glucosamine-6-phosphate)).

Inhibitors of lactonase are any known in the art.

Inhibitors of 6P gluconate dehydrogenase are any known in the art and may include, but are not limited to 6-aminonicotinamide (6AN).

Inhibitors of phosphopentose isomerase, phosphopentose epimerase, transketolase, transaldolase are any known in the art.

The pentose phosphate pathway (PPP) starts with the conversion of glucose-6-phosphate to ribose-5-phosphate; the former is obtained from the first step of glycolysis. Glycolysis accounts for a small fraction of the energy requirements of the aerobically respiring cell, the majority being obtained from oxidative phosphorylation. Despite this, the glycolytic pathway is highly active in proliferating cell masses. Glycolysis is believed act as an anabolic pathway, by providing the starting substrate for the PPP, for the ultimate synthesis of NADPH. The latter is used for biosynthetic purposes, and is highly active in proliferating cell masses. Consequently inhibition of oxidative phosphorylation pathways has the effect of inhibiting cell growth by inhibitng the supply of substrates (NADPH) to the anaboluic pathway.

### Individual and combinations of inhibitors

According to one aspect the invention, a composition comprises one or more inhibitors of aerobic ATP synthesis (*e*.*g*. inhibitors of TCA and/or oxidative phosphorylation or oxphos).
According to another aspect of the invention, a composition comprises one or more inhibitors of anaerobic ATP synthesis (*e*.*g*. inhibitors of the glycolysis directly, or indirectly through inhibition of PPP).

Owing to the properties of proliferating cells, the inventors find that a composition comprising combinations of inhibitors may also be effective at reducing a rapidly proliferating cell mass.

According to another aspect of the invention, a composition comprises one or more inhibitors of aerobic ATP synthesis and one or more inhibitors of the PPP. According to one aspect of the invention a combination of inhibitors in such a composition may be one or more inhibitors of TCA and/or oxphos and one or more inhibitors of the PPP. An inhibitor of TCA may be, for example, fluoroacetate or malonate, an inhibitor of oxphos may be oligomycin or rhodamine, and an inhibitor of the PPP may be 6-aminonicotinamide (which in the end blocks glycolysis).

According to another aspect of the invention, a composition comprises one or more inhibitors of aerobic and one or more inhibitors of anaerobic ATP synthesis. A combination may be, for example, one or more inhibitors of glycolysis and one or more inhibitors of the TCA cycle. In such case, inhibitors may be 2FDG or iodoacetate or oxamate (glycolysis) and fluoroacetate or fluorohydroxybutyrate (TCA cycle). An alternative combination may be, for example, one or more inhibitors of glycolysis and one or more inhibitors of oxidative phosphorylation. In such case, inhibitors may be 2FDG or iodoacetate or oxamate (glycolysis) and rhodamine or dinitrophenol (oxidative phosphorylation). Yet another alternative combination may be, for example, one or more inhibitors of glycolysis, one or more inhibitors of the TCA cycle and one or more inhibitors of oxidative phosphorylation. In such case, inhibitors may be 2FDG or iodoacetate or oxamate (glycolysis), fluoroacetate or fluorohydroxybutyrate (TCA cycle) and rhodamine or dinitrophenol (oxidative phosphorylation).

According to another aspect of the invention, a composition comprises one or more inhibitors of aerobic ATP synthesis, and one or more inhibitors of anaerobic ATP synthesis and/or one or more inhibitors of the PPP. Combinations of inhibited pathways include (a) TCA cycle (aerobic) and PPP (b) oxidative phosphorylation (aerobic) and PPP, (c) glycolysis (anaerobic), TCA cycle (aerobic), oxidative phosphorylation (aerobic) and PPP. In such cases combinations of inhibitors include, for example (a) fluoroacetate and 6-aminonicotinamide, (b) rhodamine and 6-aminonicotinamide, and (c) 2FDG, fluoroacetate, rhodamine and 6-aminonicotinamide.

According to another aspect of the invention, a composition comprises one or more inhibitors active against both aerobic and anaerobic ATP synthesis simultaneously. Examples of such inhibitors include, but are not limited to arsenite (blocks both glycolysis and oxphos), fluoroacetate (which blocks TCA and, as a consequence of fluorocitrate accumulation it blocks glycolysis), bromopyruvate, which blocks TCA and glycolysis, or fluoroacetoacetate (which blocks the same both cycles). According to another aspect of the invention, a composition in high concentration may reduce the bulk of restenosis by itself. In such case, the active inhibitor could be, but not limited to, 2 fluoro-D-deoxyglucose.

It is an aspect of the invention that the composition is formulated to primarily inhibit the glycolytic pathway (possibly through PPP inhibition) and secondarily inhibit either the TCA cycle or oxidative phosphorylation. Such composition is formulated according to the dose and efficacy of the inhibitory compounds.

It is possible to select which pathway of glycolysis or TCA is better to be shut down by performing a 2-FDG and a ¹¹C-acetate positron emission tomography examination, and evaluating the activity of glycolysis and TCA cycles in the said tumour, allowing to choose for each individual tumour which compound should be favoured for the inhibition.

The inventors have advantageously found that by inhibiting a combination of ATP synthetic pathways, optionally in combination with the inhibition of the PPP, the synthesis of ATP in profilerating cell masses may be most effectively inhibited, leading to rapid and selective tumour shrinkage.

The combination of ATP inhibitors is very important for the inventors. The inventor's own clinical experience (MR spectroscopy and PET-CT examinations) as well as data from the literature confirm the great variety of substrates taken up by tumours. A review of the literature data shows for instance that the affinity for 18-FDG intake varies from 3 to 100 %, depending on evaluated tumours and affected organs (*e.g*. http://www.petscaninfo.com/zportal/portals/phys/clinical/jnmpetlit/index_html/JNM_OncoA pps/JNM_Table8/article_elements_view). The inventors have also observed that a beneficial therapeutic approach is to treat several ATP synthesis pathways simultaneously.

### Derivatives

Stereoisomer, tautomers, racemates, prodrugs, metabolites, pharmaceutically acceptable salts, bases, esters, structurally related compounds or solvates of the ATP synthesis inhibitors are within the scope of the invention.

The pharmaceutically acceptable salts of the inhibitors according to the invention, *i*.*e*. in the form of water-, oil-soluble, or dispersible products, include the conventional non-toxic salts or the quaternary ammonium salts which are formed, *e*.*g*., from inorganic or organic acids or bases. Examples of such acid addition salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such a sarginine, lysine, and so forth. Also, the basic nitrogen-containing groups may be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl-bromides and others. Other pharmaceutically acceptable salts include the sulfate salt ethanolate and sulfate salts.

The term "stereoisomer", as used herein, defines all possible compounds made up of the same atoms bonded by the same sequence of bonds but having different three-dimensional structures which are not interchangeable, which the inhibitors of the present invention may possess. Unless otherwise mentioned or indicated, the chemical designation of an inhibitor herein encompasses the mixture of all possible stereochemically isomeric forms, which said compound may possess. Said mixture may contain all diastereomers and/or enantiomers of the basic molecular structure of said compound. All stereochemically isomeric forms of the inhibitors of the invention either in pure form or in admixture with each other are intended to fall within the scope of the present invention.

The inhibitors according to the invention may also exist in their tautomeric forms. Such forms, although not explicitly indicated in the inhibitors described herein, are intended to be included within the scope of the present invention.

For therapeutic use, the salts of the inhibitors according to the invention are those wherein the counter-ion is pharmaceutically or physiologically acceptable.

The term "pro-drug" as used herein means the pharmacologically acceptable derivatives such as esters, amides and phosphates, such that the resulting *in vivo* biotransformation product of the derivative is the active drug. The reference by Goodman and Gilman (The Pharmacological Basis of Therapeutics, 8th Ed, McGraw-Hill, Int. Ed. 1992, "Biotransformation of Drugs", p 13-15) describing pro-drugs generally is hereby incorporated. Pro-drugs of the compounds of the invention can be prepared by modifying functional groups present in said component in such a way that the modifications are cleaved, either in routine manipulation or in vivo, to the parent component. Typical examples of pro-drugs are described for instance in WO 99/33795, WO 99/33815, WO 99/33793 and WO 99/33792. Pro-drugs are characterized by increased bio-availability and are readily metabolized into the active inhibitors *in vivo*. Specific examples of prodrugs comprising cholesterol or vitamin A are described below.

### Slow release formulation

Described above is the provision of a top coat for regulating the release of inhibitory compounds. Another aspect of the invention relates to a composition comprising additives which control inhibitor release. According to another embodiment of the invention, the composition is a slow release formulation. Accordingly, the stent may be provided with a large or concentrated dose of inhibitor. Once the stent is at the site of treatment, inhibitor is released at a rate determined by the formulation. This avoids the need for frequently replacing stents to maintain a particular dose. Another advantage of a slow release formulation is that the composition diffuses day and night, over several days or weeks. Furthermore, the present inventors have found that inhibitor uptake can be relatively slow in some tumours by observing ¹⁸F-FDG and ¹¹C-acetate by PET-CT. Although tumours may show as an intense signal due to the sensitivity of the imaging and probe, this is deceptive of the uptake rate which can be relative low e.g in the range of ng/min. Consequently, a slowly releasing inhibitor is better able to match the rate of inhibitor take by the tumour, and avoid wasteful and toxic overdosing.

One embodiment of the present invention is a stent comprising a composition as described herein, wherein said composition further comprises one or more slow release agents. Slow release agents may be natural or synthetic polymers, or reabsorbable systems such as magnesium alloys.

Among the synthetic polymers useful according to a slow release formulation of the invention are poly(glycolic) acid, poly(lactic acid) or in general glycolic- and lactic acid based polymers and copolymers. They also include poly caprolactones and in general, poly hydroxyl alkanoates (PHAs) (poly(hydroxy alcanoic acids) = all polyester). They also include Poly (ethylene glycol), poly vinyl alcohol, poly (orthoesters), poly (anhydrides), poly (carbonates), poly amides, poly imides, poly imines, poly (imino carbonates), poly (ethylene imines), polydioxanes, poly oxyethylene (poly ethylene oxide), poly (phosphazenes), poly sulphones, lipids, poly acrylic acids, poly methylmethacrylate (PMMA), poly acryl amides , poly acrylo nitriles (Poly cyano acrylates), poly HEMA, poly urethanes, poly olefins, poly styrene, poly terephthalates, poly ethylenes, poly propylenes, poly ether ketones, poly vinylchlorides, poly fluorides, silicones, poly silicates (bioactive glass), siloxanes (Poly dimethyl siloxanes), hydroxyapatites, lactide-capronolactone, and any other synthetic polymer known to a person skilled in the art. Other synthetic polymers may be made from hydrogels based on activated polyethyleneglycols (PEGs) combined with alkaline hydrolyzed animal or vegetal proteins.

Among the natural derived polymers useful according to a slow release formulation of the invention, are poly aminoacids (natural and non natural), poly β-aminoesters. They also include poly (peptides) such as: albumines, alginates, cellulose / cellulose acetates, chitin / chitosan, collagene, fibrine / fibrinogen, gelatine, lignine. In general, proteine based polymers. Poly (lysine), poly (glutamate), poly (malonates), poly (hyaluronic acids). Poly nucleic acids, poly saccharides, poly (hydroxyalkanoates), poly isoprenoids, starch based polymers, and any other natural derived polymer known to a person skilled in the art.

Other polymers may be made from hydrogels based on activated polyethyleneglycols (PEGs) combined with alkaline hydrolyzed animal or vegetal proteins.

For both synthetic and natural polymers, the invention includes copolymers thereof are included as well, such as linear, branched, hyperbranched, dendrimers, crosslinked, functionalised (surface, functional groups, hydrophilic/hydrophobic).

The slow release composition may be formulated as liquids or semi-liquids, such as solutions, gels, hydrogels, suspensions, lattices, liposomes. Any suitable formulation known to the skilled man is within the scope the scope of the invention. According to an aspect of the invention, a composition is formulated such that the quantity of inhibitor is between less than 1% and 60 % of total slow-release polymer mass. According to an aspect of the invention, a composition is formulated such that the quantity of inhibitor is between 1% and 50%, 1% and 40%, 1% and 30%, 1% and 20%, 2% and 60%, 5% and 60%,10% and 60%, 20% and 60%, 30% and 60%, or 40% and 60% of total slow-release polymer mass.

### Solubilising agents

According to another embodiment of the present invention, the composition comprises at least one inhibitor of ATP synthesis as described herein coupled to one or more solubilising agents. Such agents change the hydrophilic and hydrophobic profile of the inhibitor, depending on the required solubility. For example, if a composition according to the invention comprises a hydrophilic TCA cycle inhibitor such as fluoroacetate, and a hydrophobic slow release polymer such as polyorthoester, the inhibitor will not adequately suspend within the composition. Similarly, a composition according to the invention comprising a very hydrophilic oxidative phosphorylation inhibitor such as rhodamine 123 and polyorthoester slow release agent, will lead to an inadequately emulisified composition. Consequently the release properties of the slow release agent may be compromised, and degradation within the body accelerated. To overcome this, the inventors have coupled at least one ATP synthesis inhibitor a solubilising agent which changes the hydrophobicity or hydrophilicity of the inhibitor, depending on the required formulation. The composition so formed is more stable. According to one aspect of the invention, the coupled compound is a prodrug wherein the solubilising agent is cleaved *in vivo*, so releasing the inhibitor. According to another aspect of the invention, the solubilising agent is cleaved from the inhibitor more rapidly by the proliferating cells.

### - Cholesterol

According to one aspect of the invention, cholesterol (II) or a derivative thereof is a solubilising agent. One embodiment of the invention is a stent provided with a composition as mentioned above in which at least one ATP synthesis inhibitor as described herein is coupled to cholesterol (III) or derivatives thereof: 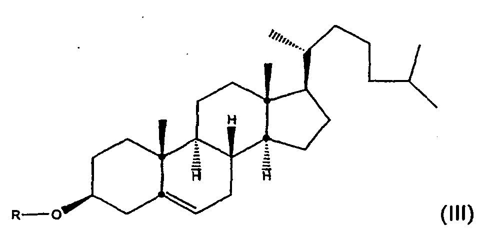 wherein R may be one of the following substances: betahydroxybutyrate, halogenated butyrate, halogenated acetate, halogenated aceto-acetate, halogenated acetamide, halogenated crotonate, halogenated acetone, halogenated citrate and halogenated oleate.

Derivatives of cholesterol are modifications which retain or enhance of activity of the parent compound. Derivatives include, but are not limited to cholesteryl-3-betahydroxybutyrate, cholesteryl-halogenated butyrate, cholesteryl-halogenated acetate, cholesteryl-halogenated aceto-acetate, cholesteryl-halogenated acetamide, cholesteryl-halogenated crotonate, cholesteryl-halogenated acetone, cholesteryl-halogenated citrate, or cholesteryl-halogenated oleate.

Halogenated means fluoro-, chloro-, bromo- or iodo-modified.

An advantage of using cholesterol or a derivative thereof as a solubilising agent is such natural metabolite can enter a cell via a number of mechanisms including through the lipid bilayer of the cell membrane. In rapidly proliferating cells, absorption is more rapid due to the requirement for cholesterol in cell membranes. Once in the lipid bilayer, flippase enzyme transfers the cholesterol-coupled inhibitor from the outer layer to the inner layer; cholesterol is intemalised in the cytosol and the inhibitor is released from cholesterol by cholesterol-metabolising enzymes.

A cholesterol is coupled to an inhibitor using known methods. For example, coupling may proceed via nucleophilic attack by electrons of an oxygen atom on the cholesterol. For example, coupling may proceed via nucleophilic attack by electrons of an oxygen atom on the inhibitor. According to another example, esters, ethers or other derivatives of cholesterol or inhibitor may be prepared to facilitate coupling. Mechanisms and knowledge of appropriate coupling moieties are known to the skilled person for the preparation of such coupled inhibitors.

One embodiment of the present invention is the composition comprises cholesteryl-fluoroacetate and polyorthoester.

### - Vitamin A

According to one aspect of the invention, vitamin A (retinol) or a derivative thereof is a solubilising agent One embodiment of the invention is a stent provided with a composition as mentioned above, wherein at least one ATP synthesis inhibitor is coupled to vitamin A or derivatives thereof. Examples of derivatives include the ether (IV) and ester (V) forms which groups facilitate ease of coupling: 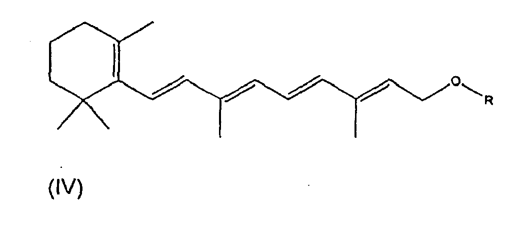 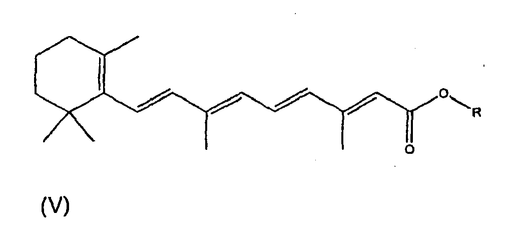 Wherein R may be one of the following substances: betahydroxybutyrate, halogenated butyrate, halogenated acetate, halogenated aceto-acetate, halogenated acetamide, halogenated crotonate, halogenated acetone, halogenated citrate, halogenated oleate.

Derivatives of vitamin A are modifications which retain or enhance of activity of the parent compound. Derivatives include, but are not limited to those mentioned above for the inhibitors and betahydroxybutyrate, halogenated butyrate, halogenated acetate, halogenated aceto-acetate, halogenated acetamide, halogenated crotonate, halogenated acetone, halogenated citrate, or halogenated oleate.

Halogenated means fluoro-, chloro-, bromo- or iodo-modified.

An advantage of using vitamin A or a derivative thereof as a solubilising agent is that such natural metabolite can enter a cell via a number of mechanisms. In rapidly proliferating cells, absorption is more rapid, especially in vitamin A metabolising cells such as found in liver tissue. The effect may be used to treat, for instance, hepatocarcinomas by injecting a slow release polymer of retinyl ether or retinoic acids ester coupled with haloacetates directly inside the hepatpcarcinoma mass. The antiproliferative effect commences once the inhibitor is liberated from the polymer and vitamin A is metabolised.

A vitamin A is coupled to an inhibitor using known methods. For example, coupling may proceed via nucleophilic attack by electrons of an oxygen atom on the vitamin A. For example, coupling may proceed via nucleophilic attack by electrons of an oxygen atom on the inhibitor. According to another example, esters or other derivatives of vitamin A or inhibitor may be prepared to facilitate coupling. Mechanisms and knowledge of active groups are known to the skilled person for the preparation of such coupled inhibitors

According to one embodiment of the present invention, the composition comprises vitamin A-fluoroacetate and polyorthoester.

### Encapsulated inhibitor

According to one aspect of the invention at least one ATP synthesis inhibitor described herein is encapsulated in one or more micro-capsules or nano-capsules.

Examples of nano-capsules (or nano-spheres) or formulations therewith include, but are not limited to a copolymer poly(ethylene oxide) with poly(L-Lactic acid) or with poly(beta-benzyl-L-aspartate); copolymer with poly(lactide-co-glycolide)-[(propylene oxide)-poly(ethylene oxide)]; polyphosphazene derivatives; poly(ethylene glycol) coated nanospheres; poly(isobutylcyanoacrylate) nanocapsules; poly(gamma-benzyl-L-glutamate)/(poly(ethylene oxide); chitosan-poly(ethylene oxide) nanoparticules; nanoparticules where the anti-proliferative drug is prepared using o-carboxymethylate chitosan (o-CMC) as wall forming material; silicone nanocapsules, solid lipid nanoparticles or nanospheres (SLNs) and any known formulation of nano-particles known to someone skilled in the art.

Examples of micro-capsules (or micro-spheres) or formulations therewith include but are not limited to multiporous beads of chitosan; coated alginate microspheres; N-(aminoalkyl) chitosan microspheres; chitosan/calcium alginate beads, poly(adipic anhydride) microspheres; gellan-gum beads; poly(D, L-lactide-co-glycolide) microspheres; alginate-poly-L-lysine microcapsules; crosslinked chitosan microspheres; chitosan/gelatin microspheres; crosslinked chitosan network beads with spacer groups; aliphatic polyesters such as 1,5-diozepan-2-one and D,L-dilactide microspheres; triglyceride lipospheres; polyelectrolyte complexes of sodium alginate chitosan; polypeptide microcapsules; albumin microspheres; and any other micro-capsule (or micro-sphere) formulation known to someone skilled in the art.

By using encapsulated inhibitor, the solubility profile of the inhibitor may be changed according to the environment of the formulation. It may thus act as a solubilising agent as mentioned above. An encapsulated inhibitor has an advantage that solubilisation does not require chemical coupling of the inhibitor. Inhibitors of ATP synthesis are generally hydrophilic while slow-release gels where present which are generally hydrophobic. Thus, an encapsulated inhibitor allows solubility within a slow-release gel, so preventing an otherwise unstable formulation.

Furthermore, encapsulation may be used to modulate release of the inhibitor (e.g. fine tune or prolong release time). Furthermore, encapsulation may be used to improve intracellular penetration, as known for encapsulations such as SLN. The advantages of encapsulated formulation may be applied to inhibitor already chemically modified to improve solubility. For example, cholesterol coupled fluoroacetate may be prepared in microcapsules within a slow release gel. The formulation so produced would provide solubility for the inhibitor, slow release modulated by the presence of capsules and active cellular penetration. Such composition may reduce the frequency and/or duration of treatment compare with conventional formulations.

One embodiment of the present invention is a stent provided with a composition as mentioned above in which at least one ATP synthesis inhibitor is encapsulated in micro- or nano-capsule(s) (or micro- or nano-sphere(s)). According to one aspect of the invention, at least one inhibitor is also pre-coupled to a solubilising agent as mentioned above.

### Sensitising agents

Another embodiment of the present invention is a stent provided with a composition as mentioned above, said composition further comprising one or more components to sensitise the proliferating cells to the inhibitors of the composition. It is achieved by unlocking or unblocking the flow into the TCA cycle. For example, where glycolysis is blocked at the level of pyruvate kinase it is possible to add serine or other elements (e.g. fructose 1-6 diP) to the composition. This forces the pyruvate kinase enzyme to adapt to its tetrameric active form and release the pyruvate kinase inhibition (Mazurek S, Lüftner D, Wechsel HW, Schneider J, Eigenbrodt E. Tumor M2-PK: a marker of the tumor metabolome, in Tumor markers: physiology, pathobiology, technology and clinical applications. Eleftherios P et al, AACC Press 2002, 471-475). The result is a stimulation of the TCA cycle. In cells where the TCA cycle is not very active, this lifts the inhibition, raises the TCA metabolism and, because of that, increase the sensitivity of the cells to TCA inhibitors.

### Dose

The size of stent and concentration of composition thereon can be calculated using known techniques by the skilled person.

According to one aspect of the invention, a stent is coated with a composition comprising TCA inhibitor such that the inhibitor concentration delivered to a subject is greater than or equal to 1, 5, 10, 20, 40, 60, 80, 100, 150, 200, 300, 400, 500, 600, 700, 800, 900, 1000 or mg inhibitor / kg, or a concentration in the range between any two of the aforementioned values. Preferably the dose is between 1 and 50 mg/kg.

According to one aspect of the invention, a stent is coated with a composition comprising glycolysis inhibitor such that the inhibitor concentration delivered to a subject is greater than or equal to 20, 40, 60, 80, 100, 150, 200, 300, 400, 500, 600, 700, 800, 900, 1000 or mg inhibitor / kg, or a concentration in the range between any two of the aforementioned values. Preferably the dose is between 100 and 1000 mg/kg.

According to one aspect of the invention, a stent is coated with a composition comprising oxidative phosphorylation inhibitor such that the inhibitor concentration delivered to a subject is greater than or equal to 5, 10, 15, 20, 25, 30, 35, 40, 45, 50, 55 or 60 mg inhibitor / kg, or a concentration in the range between any two of the aforementioned values. Preferably the dose is between 20 and 60 mg/kg.
The quantity of inhibitor per mm² of stent required to arrive at the above doses can be readily calculated by the skilled person .

The case of fluoroacetate, for example, a typical loading might be 0.1 - 2 micrograms of fluoroacetate per square mm of stent surface for a non expanded coronary stent

The case of 2FDG, for example, a typical loading might be 2 to 14, 4 to 12, 5 to 11, or 8 micrograms of 2FDG per square mm of undeployed stent surface in order to deliver an equivalent dose of 400 - 800 mg/kg dose to the first mm of vessel wall depth. The loading may be recalculated in case a different dose is required, for example for cancer, to deliver 3g/kg, the loading is calculated to be 25 microgram/square mm. Such high dosages are particularly relevant for the treatment of naive cancer patients or for the the improved treatment of cancer resection cavity, possibly using a stent coated with a membrane, allowing better surface contact of the stent surface with the area to treat Such calculations are known to the skilled person. Once in place, for instance at the level of a esophageal tumour, the patient will benefit from chemotherapy and radiotherapy, with improved efficacy.

According to an aspect of the invention, a specification combination of loading comprises less than 10 micrograms of 2FDG per square mm of undeployed coronary stent surface, together with less than 1 microgram of fluoroacetate and/or less than 5 micrograms rhodamine per square mm of undeployed stent surface, or dinitrophenol less then 5 micrograms per square mm of undeployed stent surface.

The case of bromopyruvate, for example, a typical loading might be less than 3 micrograms of bromopyruvate per square mm of undeployed stent surface in order to deliver an equivalent dose of 400 mg/kg dose or less to the first mm of vessel wall depth. Such calculations are known to the skilled person.

The case of arsenite, for example, a typical loading might be less than 3 micrograms of arsenite per square mm of undeployed stent surface in order to deliver an equivalent dose of 400 mg/kg dose or less to the first mm of vessel wall depth. Such calculations are known to the skilled person.

The case of fluoroacetoacetate, for example, a typical loading might be less than 5 micrograms of fluoroacetoacetate per square mm of undeployed stent surface in order to deliver an equivalent dose of 400 mg/kg dose or less to the first mm of vessel wall depth. Such calculations are known to the skilled person.

The case of rhodamine, for example, a typical loading might be less than 1 microgram of rhodamine per square mm of undeployed stent surface in order to deliver an equivalent dose of 200 mg/kg or less to the first mm of vessel wall depth. Such calculations are known to the skilled person. In another example, for a dose of 5mg/kg, the composition would be coated to a concentration of 0.025 micrograms rhodamine per mm² of stent strut. For the a dose of 20 mg/kg, the composition may be coated to a concentration of 0.1 micrograms rhodamine per mm² of stent strut. For a 5 micron layer of composition comprising a slow-release polymer deposited on the stent, this would represent 5 % of polymer composition, and 20 % of the total polymer amount.
According to one aspect of the invention, a stent is coated with a composition comprising rhodamine to deliver a concentration in the range 30 to 50 mg/kg, 35 to 50, and preferably 40 to 50 mg/kg. According to another aspect of the invention, a stent is coated with a composition comprising rhodamine to deliver a concentration in the range 5 to 30 mg/kg, 10 to 30, and preferably 20 to 30 mg/kg, when used in combination with cytotoxic therapy such as chemo- or radiotherapy.

According to an aspect of the invention, a specification combination of loading comprises less than 10 micrograms of iodoacetate or oxamate per square mm of undeployed coronary stent surface, together with less than 1 microgram of fluoroacetate or less than 1 microgram of fluorocitrate or less than 5 micrograms rhodamine, or less then 5 micrograms of dinitrophenol per square mm of stent surface.

The dose would be calculated according to the condition being treated. For example, a higher dose will be required for the treatment of cancer compared with vascular restenosis or stenosis. Furthermore, the dose may be lower when used in combination with other cytotoxic treatments such as chemotherapy or radiotherapy.

### Kit

A kit according to the invention may comprise at least one stent and separately, at least one composition of the present invention. The kit enables a technician or other person to coat a stent with a composition prior to insertion into a duct.

The composition, besides comprising at least one type of inhibitor of ATP synthesis, optionally at least one inhibitor of the PPP, may contain additional substances that facilitate the coating of the stent by the end-user. The composition may contain, for example, fast evaporating solvents so as to allow the rapid drying of the stent. It may contain polymeric material to allow the inhibitors to adhere to the stent and facilitate its slow release.

The composition may be applied to the stent of the kit by any means known in the art. For example, by dipping the stent in the composition, by spraying the stent with the composition, by using electrostatic forces. Such methods are known in the art.

It is an aspect of the invention that the composition is provided in a container. For example, a vial, a sachet, a screw-cap bottle, a syringe, a non-resealable vessel, a resealable vessel. Such containers are any that are suitable for containing a composition and optionally facilitating the application of the composition to the stent. Indeed, some polymers to be used for the coating and the controlled release of the active compound, such as polyorthoesthers, are extremely unstable, are very sensitive to humidity and should be conserved in a cold atmosphere and in an argon atmosphere for instance. Some active products as well, such as rotenone, are sensitive to light and heat and should be preserved in dark and cold. In such a case, a container with the composition is kept separately from the bare stent. The interventional cardiologist may open the box containing the coating and apply it on the stent just before the intervention.

A kit may comprise more than one type of stent and more than one container of composition. A kit may provide a range of stent sizes, stent configurations, stents made from different materials. A kit may provide a range of vials containing different compositions with different inhibitors, different combinations of inhibitors, different combinations of polymers. A kit may facilitate the sequential application of more than one type of composition. A kit may contain instructions for use.

### Combined radiotherapy or chemotherapy treatment

The inventors have found that the combination of a stent according to the present invention and radiotherapy and/or chemotherapy for the treatment of proliferating cells (e.g. cancerous tumours or tumour resectioned cavities) provides an effective combination therapy to shrink cellular proliferations and kill tumours or tumour cells. The use of the stent can lead to effective treatment using a fraction of the normal radiotherapy or chemotherapy therapeutic dose.

According to this aspect of the invention, a tumour present in a duct is treated using a stent as mentioned above. Alternatively, a tumour is totally or partially resected and an implant is placed inside the resection cavity as mentioned above. The site of the tumour is then treated with radiotherapy applied either from an exterior source, or by using a stent and treating the lumen from inside with brachytherapy. The combination of stent and radiotherapy treatments may lead to a rapid and effective shrinking or death of residual tumour or tumour cells.

According to another aspect of this invention, a tumour present in a duct is treated using a stent as mentioned above, and, in addition, the site of the tumour is treated with intravenous chemotherapy (for instance paclitaxel, cisplatinum, vinorelbine, etc). Alternatively, a tumour is totally or partially resected and an implant is placed inside the resection cavity as mentioned above as mentioned above, and in addition, the site of the proliferation is treated with intravenous chemotherapy.The combination of stent and radiotherapy and/or chemotherapy treatments may lead to a rapid and effective shrinking or death of residual tumour or tumour cells. It is expected that chemotherapy and/or radiotherapy will be much more efficient after the local application of the inhibitors inside the proliferating process. It is foreseen that accumulated doses of radiotherapy and/or chemotherapy could be decreased by 10 to 50 %.

One embodiment of the present invention is a stent as described herein for use in treating tumours, in combination with radiotherapy. Another embodiment of the present invention is a method for treating a tumour comprising the use of a stent as described herein in combination with radiotherapy. Another embodiment of the present invention is use of a stent for treating a tumour, in combination with radiotherapy. Another embodiment of the present invention is a stent as described herein for use in treating tumours, in combination with chemotherapy. Another embodiment of the present invention is a method for treating a tumour comprising the use of a stent as described herein in combination with chemotherapy. Another embodiment of the present invention is use of a stent for treating a tumour, in combination with chemotherapy.

Another embodiment of the present invention is a method for reducing the dose of radiotherapy treatment of a tumour, comprising applying a stent in the vicinity of said tumour prior to radiotherapy.

Another embodiment of the present invention is a method for reducing the dose of chemotherapy treatment of a tumour, comprising applying a stent in the vicinity of said tumour prior to chemotherapy.

Where radiotherapy and chemotherapy are administered, the stent of the present invention may be used to reduce both radiotherapy and chemotherapy doses. A typical chemotherapy and/or radiotherapy dose may be about 20%, 30%, 40%, 50%, 60%, 70%, 80%, 90% less than the dose normally applied to a tumour, in view of the size, location and other factors. It may be a value in the range between any two of the aforementioned values. Preferably, the dose is between 20 and 70% less than the normal dose.

Another embodiment of the present invention is a method for sensitising proliferating cells (e.g. tumour) to radiotherapy, comprising applying a stent in the vicinity of said tumour prior to radiotherapy.

Another embodiment of the present invention is a method for sensitising proliferating cells (e.g. tumour) to chemotherapy, comprising applying a stent in the vicinity of said tumour prior to chemotherapy.

According to one aspect of the invention, the stent is applied to a subject at least 8 hours, 10 hours, 12 hours, 14 hours, 16 hours, 18 hours, 20 hours, 22 hours, 1 day, 2 days, 3 days, 4 days, 6, days, 8 days, 10 days, 12 days, 14 days, 3 weeks or 4 weeks before the start of radio- and/or chemotherapy, or for a period between any two of the aforementioned periods. Preferably, the stent is in place for between 12 hours to 4 weeks before commencement of radio- and/or chemotherapy.

### EXAMPLES

The invention is illustrated by the following non-limiting examples. They illustrate the effectiveness of a selection of glycolysis, TCA cycle, oxidative phosphorylation inhibitors described above. The inihibitory properties of the other inhibitors not mentioned in the examples are known, and the skilled person may readily substitute the exemplified inhibitors with equivalent pathway inhibitors such as listed above.

### EXAMPLE 1

A composition comprising between 1 and 10 micrograms of 2-fluoro-deoxyglucose, 0.1 to 5 micrograms of fluoroacetate, and 1 to 10 micrograms of rhodamine per square mm of undeployed stent and a suitable polymer is coated onto a balloon inflatable stent. The stent is introduced into a subject suffering from localised vascular stenosis using the percutaneous, transluminal, coronary angioplasty (PTCA) intervention. Six months after the intervention, an angiography is made of the area of the intervention. The degree of restenosis is calculated as a function of the percentage of patent vessel lumen.

### EXAMPLE 2 (Comparative)

A composition comprising between 10 and 20 micrograms of bromopyruvate per square mm of undeployed stent and a suitable polymer is coated onto a balloon inflatable stent. The stent is introduced into a subject suffering from localised vascular stenosis using the percutaneous, transluminal, coronary angioplasty (PTCA) intervention. Six months after the intervention, an angiography is made of the area of the intervention. The degree of restenosis is calculated as a function of the percentage of patent vessel lumen.

### EXAMPLE 3 (Comparative)

A composition comprising between 0.1 and 10 micrograms of arsenite per square mm of undeployed stent and a suitable polymer is coated onto a balloon inflatable stent. The stent is introduced into a subject suffering from localised vascular stenosis using the percutaneous, transluminal, coronary angioplasty (PTCA) intervention. Six months after the intervention, an angiography is made of the area of the intervention. The degree of restenosis is calculated as a function of the percentage of patent vessel lumen.

### EXAMPLE 4 (Comparative)

A composition comprising between 1 and 10 micrograms of dinitrophenol plus 1 to 20 micrograms of bromopyruvate per square mm of undeployed stent and a suitable polymer is coated onto a balloon inflatable stent. The stent is introduced into a subject suffering from localised vascular stenosis using the percutaneous, transluminal, coronary angioplasty (PTCA) intervention. Six months after the intervention, an angiography is made of the area of the intervention. The degree of restenosis is calculated as a function of the percentage of patent vessel lumen.

### EXAMPLE 5 (Comparative)

A composition comprising 1 to 10 micrograms fluoroacetate per square mm of undeployed stent and a suitable polymer is coated onto a self-expandable stent. The stent is introduced into a subject suffering from localised vascular stenosis using the percutaneous, transluminal, coronary angioplasty (PTCA) intervention. Six months after the intervention, an angiography is made of the area of the intervention. The degree of restenosis is calculated as a function of the percentage of patent vessel lumen

### EXAMPLE 6 (Comparative)

A composition comprising 1 to 10 micrograms fluoroacetoacetate per square mm of undeployed stent and a suitable polymer is coated onto a self-expandable stent. The stent is introduced into a subject suffering from localised vascular stenosis using the percutaneous, transluminal, coronary angioplasty (PTCA) intervention. Six months after the intervention, an angiography is made of the area of the intervention. The degree of restenosis is calculated as a function of the percentage of patent vessel lumen

### EXAMPLE 7

A composition comprising between 1 and 10 micrograms of acetoacetate (ketone body) and 1 to 10 micrograms of rhodamine per square mm of undeployed stent and a suitable polymer is coated onto a balloon inflatable stent. The stent is introduced into a subject suffering from localised vascular stenosis using the percutaneous, transluminal, coronary angioplasty (PTCA) intervention. Six months after the intervention, an angiography is made of the area of the intervention. The degree of restenosis is calculated as a function of the percentage of patent vessel lumen.

### EXAMPLE 8

A composition comprising between 1 and 10 micrograms of betahydroxybutyrate (ketone body) and 1 to 10 micrograms of rhodamine per square mm of undeployed stent and a suitable polymer is coated onto a balloon inflatable stent. The stent is introduced into a subject suffering from localised vascular stenosis using the percutaneous, transluminal, coronary angioplasty (PTCA) intervention. Six months after the intervention, an angiography is made of the area of the intervention. The degree of restenosis is calculated as a function of the percentage of patent vessel lumen.

### EXAM PLE 9 (Comparative)

A composition comprising between 1 and 10 micrograms of oxamate and 1 to 10 micrograms of dinitrophenol per square mm of undeployed stent and a suitable polymer is coated onto a balloon inflatable stent. The stent is introduced into a subject suffering from localised vascular stenosis using the percutaneous, transluminal, coronary angioplasty (PTCA) intervention. At monthly periods after the intervention, an angiography is made of the area of the intervention. The degree of restenosis is calculated as a function of the percentage of patent vessel lumen.

### EXAMPLE 10

A nitinol or phynox self-expanding stent is coated with a thin foil of Dacron on which a 1 to 2 mm layer of hydrogel is placed. This hydrogel layer may be made from a mixture of PEG and from soya proteins in order to retain a maximal amount of water. It can also be a polymer such as a polyorthoesther.
The polymer is saturated with FDG (or oxamate) and Rhodamine. For instance 100 mg of FDG per g of polymer (or 200 mg of oxamate per g of polymer) and 100 mg of rhodamine per g of polymer are used.
The 6 cm long coated nitinol stent is placed inside the oesophagus, in the centre of an area where a 3 cm long oesophageal tumor is present. This stent is placed 12 hours to several days before a chemotherapy and/or a radiation therapy regimens is started.
The active stent diffuses FDG (or oxamate) alone combined with Rhodamine during several days and saturates the oesophageal cancer tissue absorbing both substances actively. These substances block the glycolysis and the oxphos mechanisms in the tumour cells, rendering the replicating cells very sensitive to any therapeutic agent (chemotherapy or radiation therapy). FDG and Rhodamine also diffuse from the saturated tumour to the regional draining tumoral lymphnodes, rendering the tumour cells located inside the lymphnodes very sensitive to chemotherapy and/or radiotherapy as well.

The patient is given one of the standard chemotherapeutic regimens known today to treat oesophageal cancer: Taxotere 20 mg/m²/week one day a week for 4 weeks together with 5-FU, 300 mg/m2/24h continuous infusion during 5 days during week 1 and 4. Radiation therapy is delivered during 4 weeks, giving 40 Gy in 2 Gy fractions, one fraction a day. At least 80 % of patients are free from disease and may either undergo oesophageal resection or avoid surgical intervention if disappearance of the tumour is shown by imaging procedures and repeated biopsies. The rate of complete tumour response with this regimen is above 80 %, while only two thirds of standard radiation is delivered (40-45 Gy instead of 70 Gy) to cure oesophageal cancer. Such high sterilization rates have never been achieved before, as, with the most potent combinations of chemotherapy and radiation therapy until now, maximal sterilization rates of 50 % are achieved.

### EXAMPLE 11

A similar therapy may be performed in rectal cancer. Rectal cancer is usually treated by chemotherapy and radiation therapy before surgery in order to increase the rate of success of total resection, to increase the probability of sphincter sparing surgery, and to reduce local tumour recurrences.
A polylactic acid knitted resorbable stent is mixed with FDG and Rhodamine (or is coated as in the former example of oesophageal tumour), or a nitinol stent is coated with a membrane and a polymer as described in Example 11. Similar concentrations of both products are placed inside or on the stent. The stent is introduced inside the rectum using standard rectoscopy procedures and is placed in such a way that it covers the area where the tumour ulcer is present, allowing the active substance to diffuse mainly at the level
where the lesion is located. The stent may be totally coated with an active membrane, or it may be partially coated, for instance on 50 % of its surface. This allows limiting the deposition of the active substance(s) only at the level of the tumour area.
The patient is treated with standard chemotherapy infusion, capecitabine 825 mg/m² every 12 hours for 5 days per week, and receives up to 46 Gy of radiotherapy in 23 fractions on the pelvis, followed by surgery. Pathological complete response is achieved in more than 30 % of patients, much better than the usual rate 10 % complete response rate. The expected rate of recurrences is much lower than 10 to 20 % of local recurrences seen without any preoperative therapy.

### EXAMPLE 12

A female patient presents with a uterine cancer with a limited mural invasion depth (less than half of uterine wall thickness). The moderate invasion of the uterine muscle wall has been established by performing a standard high field magnetic resonance (MR) examination (at 1.5 Tesla). No pathologic lymphnodes in the pelvic draining areas were seen on this examination. The diagnosis of endometrial cancer has been established by taking a histological sample (aspiration with a Cornier tube). It is concluded that the patient presents with a localized cancer disease of the uterus.
The patient would like to avoid or postpone a surgical intervention. A self expandable coated stent, optionally biodegradable, coated with a 2 to 3 mm layer of polymer (hydrogel or other) is inserted inside the uterus after a dilatation of the cervical canal has been performed. The length of the chosen stent depends on the hysterometry and on the intra-uterine length, as seen by MR. This length may vary between 2 and 10 cm. The polymeric coating contains 200 mg of oxamate per g of polymer, and 100 mg of Rhodamine per g of polymer. The release of the drugs lasts for 1 to 3 weeks. After this period, the patient undergoes three sessions of intra-uterine brachytherapy and receives three exposures at 7 Gy prescribed at 1 cm from the endometrial mucosa surface by using a brachytherapy endo-uterine applicator (endo-uterine tube). The brachytherapy treatment is performed once a week for three successive weeks. The stent is retrieved and the patient is observed during following years. The recurrence rate is below 10 %. When a recurrence or a second endouterine cancer is detected, a uterus resection remains possible because the treatment was strictly limited to the uterine wall and because the brachytherapy doses were low (approximately half of the standard doses). Such a limited treatment will allow reducing the need for surgical interventions in a significant amount of patients.

### EXAMPLE13 (Comparative)

A 80 year old patient is referred with a 1 cm in diameter squamous cell carcinoma of the outer auditive canal. A stent coated with a polymeric membrane (see above), which contains 20 % per polymeric weight of iodoacetate and 10 % per polymeric of weight of dinitrophenol is inserted inside the auditive canal. The tumour is impregnated for between 12 hours to several days by the active substances before the start of radiotherapy. 40 to 50 Gy is delivered on the auditive canal by a 9 MeV electron beam, 2 Gy per fraction, five fractions a week. A total remission is achieved and the patient is followed up. The insertion of the coated stent has avoid the standard 20 Gy boost on the auditive canal to reach a therapeutic dose of 70 to 75 Gy, and, therefore, has reduced the level of toxicity.

### EXAMPLE 14 (Comparative)

A 65 year old women is addressed with a vaginal cancer, 3 cm in diameter, located on the left side of the vagina and 2.5 cm below the vaginal fornix. A self-expanding stent coated with a polymeric membrane is inserted and placed in contact with the tumour area. The membrane contains 3-halo-pyruvate at a concentration of 20 % of the polymer and 10 % per polymer of rotenone. The tumour is left in contact with the stent for 12 hours to several days. The patient is thereafter treated by external radiotherapy to the vagina and to the lymphatic areas at a dose of 40 to 45 Gy. A boost by 10 to 15 Gy of brachytherapy is delivered inside the vagina. The combined therapy allows a reduction in the standard dose of radiation by 10 to 20 Gy, while keeping the same therapeutic efficiency. The treatment is relatively non-toxic and the local control rate is reached in more than 90 % of patients.

### EXAMPLE 15 (Comparative)

A 55 year old patient is referred with a 3 cm long oesophageal cancer located 30 cm from the superior dental arch. A stent as described in Example 11 is implanted at the site of the tumor using a self-expandable stent. The polymer contains 10 to 100 mg per g of polymer of bromopyruvate and 5 mg of Dinitrophenol per gm of polymer. The stent is left in place for between 12 to 48 hours before a standard radiation and chemotherapy regimen for oesophageal cancers is undertaken (see Example 11). When implemented such patients reach 60 to 80 % of total tumour shrinkage, higher than what is seen today. The impact on overall survival is evaluated on follow-up.

### EXAMPLE 16

Human smooth muscle cells (SMC) were treated with rhodamine123 and/or with FDG.

Three levels of Rhodamine123 concentration were used first: 0.05, 1 and 2.5 mg/ml. With these concentrations, the proliferation rates of SMC were respectively 90 + 21.6 %, 51.2 + 10.7 %, and 7.3 + 5.1 % if compared to non rhodamine treated cells. With higher rhodamine concentrations, the SMC proliferation was totally suppressed.

A combined composition of FDG and Rhodamine at various dosages were evaluated on the same human SMC cultures. The doses (FDG/rhodamine in mg/ml) were as follows:
0.05/0.01, 0.1/0.05, 0.25/0.1, 0.5/0.25, 0.75/0.5 (low dose combinations);
50/25, 50/50, 75/25, 75/50, 100/25, 100/50 (higher dose concentrations).
At the lowest dose combinations, the cell proliferation fell down to 60 % of the control cell values at the higher concentrations chosen.
When the higher concentrations combination were taken, the SMC proliferation fell to 0 % at all concentrations, showing the high efficacy of the combination.

### EXAMPLE 17

A 70 year old female patient presents with a 1 cm ductal breast cancer located in the upper outer quadrant of the right breast. She undergoes sentinel node dissection. No pathologic lymphnode is found on this examination. A tumorectomy is performed and a biodegradable stent is inserted inside the resection cavity. The stent may be a 1- 5 cm diameter and 2 to 7 cm long biodegradable metallic stent with thin struts, made from a biodegradable magnesium alloy and coated with a 2 to 3 mm polymer sheet (hydrogel or other) which contains the active substances. Alternatively, the stent may be made from a resorbable polymer (lactide and capronolactone for instance), with thick struts impregnated with active substances: 20-25 % of polymer material being made from 2FDG; or 15 % FDG and 15 % Rhodamine; or 10 % 2FDG, 10 % rhodamine and 5 % fluoroacetate.
When stent is used with FDG alone, the patient undergoes external radiotherapy to the breast, or brachytherapy inside the stent while FDG is still eluting. The recurrence rate in the resection scar walls at 10 years is inferior to 5 %.
When the active substances of the stent include FDG, Rhodamine 123 and fluoroacetate, the patient may avoid external or internal radiotherapy. The recurrence rate in the resection scar wall is in the range of 1-4 %. The patient may receive hormone treatment simultaneously.

### EXAMPLE 18

A coronary stent made from a magnesium alloy is coated with a 5 microns lactide-capronolactone polymer layer. The stent, once opened, will have a diameter of 3.5 mm. The volume of tissue to be treated, per centimeter of stent length, once the stent is deployed, is 150 mm³ (considering the vessel wall thickness of 1 mm), or 0.15 ml. We have shown that 5 microg/ml of rhodamine are enough to stop smooth muscle cell proliferation, which means, that per cm of stent length, 1 microg of Rhodamine 123 is enough to prevent restenotic proliferation. If polymer layer is 5 microns thick, Rhodamine 123 may be present at a 20 % concentration inside the polymer, or 1 microg/mm2. The stent is introduced into a subject suffering from localised vascular stenosis using the percutaneous, transluminal, coronary angioplasty (PTCA) intervention. 6 months after the intervention, an angiography is made of the area of the intervention. The degree of restenosis is calculated as a function of the percentage of patent vessel lumen.

## Claims

1. A medical stent provided with a composition comprising an inhibitor of oxidative phosphorylation that is rhodamine 123.

2. A kit comprising a) at least one medical stent and b) a composition comprising an inhibitor of oxidative phosphorylation that is rhodamine 123.

3. Use of a composition comprising an inhibitor of oxidative phosphorylation that is rhodamine 123, for the preparation of a composition for providing a medical stent for inhibiting cell proliferation via inhibition of the oxidative phosphorylation pathway.

4. Use of a composition comprising an inhibitor of oxidative phosphorylation that is rhodamine 123, for the preparation of a composition for providing a medical stent for sensitizing proliferating cells to treatment by radiotherapy.

5. Use of a composition comprising an inhibitor of oxidative phosphorylation that is rhodamine 123, for the preparation of a composition for providing a medical stent for sensitizing proliferating cells to treatment by chemotherapy.

6. Stent, use or kit according to any of claims 1 to 5, wherein said stent is provided with one or more cavities for containing and releasing said composition.

7. Stent, use or kit according to any of claims 1 to 6, wherein said stent is at least partly made from a material which is biodegradable *in situ*.

8. Stent, use or kit according to any of claims 1 to 7, wherein said stent comprises a magnesium based alloy.

9. Stent, use or kit according to any of claims 1 to 8, wherein said stent is at least partly made from a material which is non-biodegradable *in situ* and is impregnated with said composition.

10. Stent, use or kit according to any of claims 1 to 9, wherein said stent is at least partly provided with said composition.

11. Stent, use or kit according to any of claims 1 to 10, wherein said composition further comprises one or more slow release agents to facilitate slow release of inhibitor.

12. Stent, use or kit according to any of claim 11, wherein said slow release agent is any of magnesium alloys, poly(glycolic) acid, poly(lactic acid) or in general glycolic- and lactic acid based polymers, copolymers, poly caprolactones and in general, poly hydroxyl alkanoate,s poly(hydroxy alcanoic acids), Poly (ethylene glycol), poly vinyl alcohol, poly (orthoesters), poly (anhydrides), poly (carbonates), poly amides, poly imides, poly imines, poly (imino carbonates), poly (ethylene imines), polydioxanes, poly oxyethylene (poly ethylene oxide), poly (phosphazenes), poly sulphones, lipids, poly acrylic acids, poly methylmethacrylate, poly acryl amides, poly acrylo nitriles (Poly cyano acrylates), poly HEMA, poly urethanes, poly olefins, poly styrene, poly terephthalates, poly ethylenes, poly propylenes, poly ether ketones, poly vinylchlorides, poly fluorides, silicones, poly silicates (bioactive glass), siloxanes (Poly dimethyl siloxanes), hydroxyapatites, lactide-capronolactone, natural and non natural poly aminoacids , poly β-aminoesters, albumines, alginates, cellulose / cellulose acetates, chitin / chitosan, collagene, fibrine / fibrinogen, gelatine, lignine, proteine based polymers, Poly (lysine), poly (glutamate), poly (malonates), poly (hyaluronic acids), Poly nucleic acids, poly saccharides, poly (hydroxyalkanoates), poly isoprenoids, starch based polymers, copolymers thereof, linear, branched, hyperbranched, dendrimers, crosslinked, functionalised derivatives thereof, or hydrogels based on activated polyethyleneglycols combined with alkaline hydrolyzed animal or vegetal proteins.

13. Stent, use or kit according to any of claims 1 to 12, wherein at least one inhibitor is encapsulated in a micro-capsule or nano-capsule.

14. Stent, use or kit according to claim 13 wherein a nano-capsule comprises any of a copolymer poly(ethylene oxide) with poly(L-Lactic acid) or with poly(beta-benzyl-L-aspartate); copolymer with poly(lactide-co-glycolide)-[(propylene oxide)-poly(ethylene oxide)]; polyphosphazene derivatives; a poly(ethylene glycol) coated nanosphere; a poly(isobutylcyanoacrylate) nanocapsules poly(gamma-benzyl-L-glutamate)/(poly(ethylene oxide); chitosan-poly(ethylene oxide) nanoparticules; o-carboxymethylate chitosan, or a solid lipid nanosphere (SLN).

15. Stent, use or kit according to claim 13 wherein a micro-capsule is a micro-capsule comprises any of chitosan; a coated alginate microsphere; an N-(aminoalkyl) chitosan microsphere; a chitosan/catcium alginate bead, a poly(adipic anhydride) microsphere; a gellan-gum bead; a poly(D, L-lactide-co-glycolide) microsphere; an alginate-poly-L-lysine microcapsule; a crosslinked chitosan microsphere; a chitosan/gelatin microsphere; a crosslinked chitosan network bead with spacer groups; an aliphatic polyester; a 1,5-diozepan-2-one microsphere; a D,L-dilactide microsphere; a triglyceride liposphere; a polyelectrolyte complexe of sodium alginate chitosan; a polypeptide microcapsule, oran albumin microsphere.

16. Stent, use or kit according to any of claims 1 to 15, said rhodamine 123 is coupled to solubilising agent.

17. Stent, use or kit according claim 16, wherein said solubilising agent is a solubilizing agent is cholesterol or derivative thereof.

18. Stent, use or kit according to claim 17, wherein said cholesterol derivatives are any of cholesteryl-3-betahydroxybutyrate, cholesteryl-halogenated butyrate, cholesteryl-halogenated acetate, cholesteryl-halogenated aceto-acetate, cholesteryl-halogenated acetamide, cholesteryl-halogenated crotonate, cholesteryl-halogenated acetone, cholesteryl-halogenated citrate, or cholesteryl-halogenated oleate.

19. Stent, use or kit according to claim 17, wherein said solubilising agent is vitamin A or derivative thereof.

20. Stent, use or kit according to claim 19, wherein derivative of vitamin A is formula (IV) or (V): 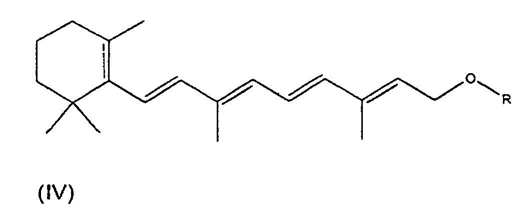 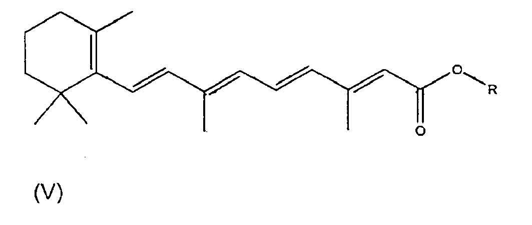 wherein R is selected from the group consisting of betahydroxybutyrate, halogenated butyrate, halogenated acetate, halogenated aceto-acetate, halogenated acetamide, halogenated crotonate, halogenated acetone, halogenated citrate, and halogenated oleate.

21. Stent, use or kit according to any of claims 1 to 20 wherein the rhodamine 123 is present in a quantity to inhibit the oxidative phosphorylation pathway.

22. Stent, use or kit according to any of claims 1 to 21 wherein said stent is provided with an amount of composition to deliver a concentration between 10 and 60 mg rhodamine 123 / kg.

23. Stent, use or kit according to any of claims 1 to 22 wherein said composition further comprises one or more polymers to facilitate attachment of the composition to the stent and/or slow release of the composition.

24. Stent, use or kit according to claim 23, wherein said polymer is one or more of:
- aliphatic polyesters, poly(amino acids), copoly(ether-esters), polyalkylenes oxalates, polyamides, poiy(iminocarbonates), polyanhydrides, polyorthoesters, polyoxaesters, polyamidoesters, polylactic acid, polyethylene oxide, polycaprolactone, polyhydroxybutyrate valerates, polyoxaesters containing amido groups, poly(anhydrides), polyphosphazenes, silicones, biomolecules and blends thereof,
- lactic acid D-,L- and meso lactide, epsilon -caprolactone, glycolide glycolic acid, hydroxybutyrate, hydroxyvalerate, para-dioxanone, trimethylene carbonate and its alkyl derivatives, 1,4-dioxepan-2-one, 1,5-dioxepan-2-one, 6,6-dimethyl-1,4-dioxan-2-one and polymer blends thereof,
- polyanhydrides from diacids of the form HOOC-C₆H₄-O-(CH₂)ₘ-O-C₆H₄-COOH wherein m is an integer in the range of from 2 to 8 and copolymers thereof with aliphatic alpha-omega diacids of up to 12 carbons,
- naturally enzymatically or are hydrolytically unstable polymers, fibrin, fibrinogen, collagen, gelatin, glycosaminoglycans, elastin, and absorbable biocompatible polysaccharides such as chitosan, starch, fatty acids and esters thereof, glucoso-glycans and hyaluronic acid,
- hydrogels
- polyurethanes, silicones, poly(meth)acrylates, polyesters, polyalkyl oxides, polyethylene oxide, polyvinyl alcohols, polyethylene glycols and polyvinyl pyrrolidone, as well as, hydrogels, hydrogels from crosslinked polyvinyl pyrrolidinone and polyesters, polyolefins, polyisobutylene and ethylene-alphaolefin copolymers, acrylic polymers, methacrylate and copolymers, vinyl halide polymers and copolymers, polyvinyl chloride, polyvinyl ethers, polyvinyl methyl ether, polyvinylidene polyvinylidene fluoride polyvinylidene chloride, polyacrylonitrile, polyvinyl ketones, polyvinyl aromatics, polystyrene, polyvinyl esters, polyvinyl acetate, copolymers of vinyl monomers with each other and olefins, etheylene-methyl methacrylate copolymers, acrylonitrile-styrene copolymers, ABS resins and ethylene-vinyl acetate copolymers, polyamides, Nylon 66 and polycaprolactam, alkyd resins, polycarbonates, polyoxymethylenes, polyimides, polyethers, epoxy resins, polyurethanes, rayon, rayon-triacetate, cellulose, cellulose acetate, cellulose acetate butyrate, cellophane, cellulose nitrate, cellulose propionate, cellulose ethers, carboxymethyl cellulose and hydoxyalkyl celluloses, and combinations thereof,
- polyamides of the form-NH-(CH₂)n-CO- and NH-(CH₂)x-NH-CO-(CH₂)y-CO, wherein n is preferably an integer in from 6 to 13; x is an integer in the range of form 6 to 12; and y is an integer in the range of from 4 to 16,
- bioabsorbable elastomers, aliphatic polyester elastomers,
- elastomeric copolymers of epsilon-caprolactone and glycolide, preferably having a mole ratio of epsilon -caprolactone to glycolide of from about 35:65 to about 65:35, more preferably 45:55 to 35:65,
- elastomeric copolymers of epsilon-caprolactone and lactide, L-lactide, D-lactide blends thereof or lactic acid copolymers, preferably having a mole ratio of epsilon -caprolactone to lactide of from about 35:65 to about 90:10 and more preferably from about 35:65 to about 65:35 and most preferably from about 45:55 to 30:70 or from about 90:10 to about 80:20,
- elastomeric copolymers of p-dioxanone and lactide including L-lactide, D-lactide and tactic acid, preferably having a mole ratio of p-dioxanone to lactide of from about 40:60 to about 60:40,
- elastomeric copolymers of epsilon-caprolactone and p-dioxanone, preferably having a mole ratio of epsilon-caprolactone to p-dioxanone of from about 30:70 to about 70:30,
- elastomeric copolymers of p-dioxanone and trimethylene carbonate, preferably having a mole ratio of p-dioxanone to trimethylene carbonate of from about 30:70 to about 70:30,
- elastomeric copolymers of trimethylene carbonate and glycolide, preferably having a mole ratio of trimethylene carbonate to glycolide of from about 30:70 to about 70:30,
- elastomeric copolymer of trimethylene carbonate and lactide including L-lactide, D-lactide, blends thereof or lactic acid copolymers, preferably having a mole ratio of trimethylene carbonate to lactide of from about 30:70 to about 70:30 and blends thereof.

25. Stent or kit according to any of claims 1 to 24 suitable for use in inhibiting cell proliferation.

26. Stent, use or kit according to any of claims 1 to 25, wherein said cell proliferation is restenosis or stenosis.

27. Stent, use or kit according to any of claims 1 to 26, wherein said cell proliferation is cancer.

28. Stent, use or kit according to any of claims 1 to 27 wherein the stent is placed in an artery or vein.

29. Stent, use or kit according to any of claims 1 to 27 wherein the stent is placed in a bronchial duct.

30. Stent, use or kit according to any of claims 1 to 27, wherein the stent is placed in a digestive duct such as esophagus, bowel, and/or biliary tract.

31. Stent, use or kit according to any of claims 1 to 27, wherein the stent is placed in a gynecological duct such as uterus, cervix, and/or vagina.

32. Stent, use or kit according to any of claims 1 to 27, wherein the stent is placed in a resection cavity or scar.

33. Stent, use or kit according to any of claims 1 to 32, wherein said rhodamine is a derivative of rhodamine.

34. A medical stent at least partly covered with an expandable membrane over the expandable region, said membrane provided with a composition comprising rhodamine 123.

35. A medical stent according to claim 34 wherein said membrane is biodegradable or non biodegradable material.

36. A medical stent according to claims 34 or 35 wherein said membrane is at least partly formed from any of aliphatic polyester copolymers, elastomeric copolymers of epsilon - caprolactone and glycolide, elastomeric copolymers of E-caprolactone and lactide, copolymers of E-caprolactone and lactic acid, elastomeric copolymers of p-dioxanone and lactide, elastomeric copolymers of p-dioxanone and lactic acid, elastomeric copolymers of epsilon-caprolactone and p-dioxanone, elastomeric copolymers of p-dioxanone and trimethylene carbonate, elastomeric copolymers of trimethylene carbonate and glycolide, elastomeric copolymer of trimethylene carbonate and lactide, or elastomeric copolymer of trimethylene carbonate and lactic acid.

37. A medical stent according to any of claims 34 to 36, wherein said stent is at least partly made from a material which is biodegradable *in situ* and is impregnated with said composition.

38. A medical stent according to any of claims 34 to 37, wherein said stent comprises a magnesium based alloy.

39. A medical stent according to any of claims 34 to 38, wherein said stent is at least partly made from a material which is non-biodegradable.

40. A medical stent according to any of claims 34 to 39, wherein said membrane is at least partly provided with said composition.

41. Use of a composition comprising rhodamine 123, for the preparation of a composition for providing a medical stent at least partly covered with an expandable membrane for inhibiting cell proliferation.

42. Use of a composition comprising rhodamine 123 for the preparation of a composition for providing a medical stent at least partly covered with an expandable membrane for sensitizing proliferating cells to radiotherapy.

43. Use of a composition comprising rhodamine 123 for the preparation of a composition for providing a medical stent at least partly covered with an expandable membrane for sensitizing a proliferating cells to chemotherapy.

44. A kit comprising a) at least one medical stent at least partly covered with an expandable membrane and b) a composition comprising rhodamine 123.

45. A medical stent, use or kit according to any of claims 34 to 44, wherein said composition further comprises one or more slow release agents to facilitate slow release of inhibitor.

46. A medical stent, use or kit according to claim 45, wherein said slow release agent is any as defined in claim 12.

47. A medical stent, use or kit according to any of claims 34 to 46, wherein said rhodamine 123 is encapsulated a micro-capsule or nano-capsule.

48. A medical stent, use or kit according to claim 47 wherein a nano-capsule is a nano-capsule as defined in claim 14.

49. A medical stent according to claim 48 wherein a micro-capsule is a micro-capsule as defined in claim 15.

50. A medical stent, use or kit according to any of claims 34 to 49, wherein said rhodamine 123 is coupled to solubilising agent.

51. A medical stent, use or kit according to claim 50, wherein said solubilising agent is a solubilizing agent as defined in any of claims 17 to 20.

52. A medical stent, use or kit according to any of claims 34 to 51, wherein said composition further comprises one or more polymers to facilitate attachment of the composition to the membrane and/or slow release of the composition.

53. A medical stent, use or kit according to claim 52, wherein said polymer is one or more of the polymers as defined in claim 23.

54. A medical stent or a kit according to any of claims 34 to 40 and 44 to 53, suitable for use in inhibiting cell proliferation.

55. A medical stent or kit or a use according to any of claims 41 to 54, , wherein said cell proliferation is restenosis or stenosis.

56. A medical stent, a kit or a use according to any of claims 41 to 54, wherein said cell proliferation is cancer.

57. A medical stent, a kit or a use according to any of claims 34 to 56 wherein said stent is placed in an artery or vein.

58. A medical stent, a kit or a use according to any of claims 34 to 56 wherein said stent is placed in a bronchial duct.

59. A medical stent, a kit or a use according to any of claims 34 to 56, wherein said stent is placed in a digestive duct such as esophagus, bowel, and/or biliary tract.

60. A medical stent, a kit or a use according to any of claims 34 to 56, wherein said stent is placed in a gynecological duct such as uterus, cervix, and/or vagina.

61. A medical stent, a kit or a use according to any of claims 34 to 56, wherein said stent is placed in a malignant or benign resection cavity or scar.

62. Stent, use or kit according to any of claims 34 to 61, wherein said rhodamine is a derivative of rhodamine.

## Patentansprüche

1. Medizinischer Stent, der mit einer Zusammensetzung bereitgestellt ist, die einen Inhibitor einer oxidativen Phosphorylierung umfasst, der Rhodamin 123 ist.

2. Kit, umfassend: a) mindestens einen medizinischen Stent und b) eine Zusammensetzung, die einen Inhibitor einer oxidativen Phosphorylierung umfasst, der Rhodamin 123 ist.

3. Verwendung einer Zusammensetzung, die einen Inhibitor einer oxidativen Phosphorylierung umfasst, der Rhodamin 123 ist, in der Herstellung einer Zusammensetzung zur Bereitstellung eines medizinischen Stents zur Hemmung einer Zellproliferation durch Hemmung des oxidativen Phosphorylierungspfades.

4. Verwendung einer Zusammensetzung, die einen Inhibitor einer oxidativen Phosphorylierung umfasst, der Rhodamin 123 ist, in der Herstellung einer Zusammensetzung zur Bereitstellung eines medizinischen Stents zur Sensibilisierung proliferierender Zellen gegenüber einer Behandlung durch Strahlentherapie.

5. Verwendung einer Zusammensetzung, die einen Inhibitor einer oxidativen Phosphorylierung umfasst, der Rhodamin 123 ist, in der Herstellung einer Zusammensetzung zur Bereitstellung eines medizinischen Stents zur Sensibilisierung proliferierender Zellen gegenüber einer Behandlung durch Chemotherapie.

6. Stent, Verwendung oder Kit nach einem der Ansprüche 1 bis 5, wobei der Stent mit einem oder mehreren Hohlräumen zur Aufnahme und Freisetzung der Zusammensetzung bereitgestellt ist.

7. Stent, Verwendung oder Kit nach einem der Ansprüche 1 bis 6, wobei der Stent mindestens teilweise aus einem Material besteht, das in situ biologisch abbaubar ist.

8. Stent, Verwendung oder Kit nach einem der Ansprüche 1 bis 7, wobei der Stent eine Legierung auf Magnesiumbasis umfasst.

9. Stent, Verwendung oder Kit nach einem der Ansprüche 1 bis 8, wobei der Stent mindestens teilweise aus einem Material besteht, das in situ nicht biologisch abbaubar ist und mit der Zusammensetzung imprägniert ist.

10. Stent, Verwendung oder Kit nach einem der Ansprüche 1 bis 9, wobei der Stent mindestens teilweise mit der Zusammensetzung bereitgestellt ist.

11. Stent, Verwendung oder Kit nach einem der Ansprüche 1 bis 10, wobei die Zusammensetzung des Weiteren ein oder mehr langsame Freisetzungsmittel umfasst, um ein langsames Freisetzen des Inhibitors zu erleichtern.

12. Stent, Verwendung oder Kit nach Anspruch 11, wobei das langsame Freisetzungsmittel eines ist von Magnesiumlegierungen, Poly(glycolsäure) oder im Allgemeinen Polymeren auf Glycol- und Milchsäurebasis, Copolymeren, Polycaprolactonen und im Allgemeinen Polyhydroxylalkanoaten, Poly(hydroxyalkansäuren), Poly-(ethylenglycol), Polyvinylalkohol, Poly(orthoestern), Poly(anhydriden), Poly(karbonaten), Polyamiden, Polyimiden, Polyiminen, Poly(iminokarbonaten), Poly(ethyleniminen), Polydioxanen, Polyoxyethylen(polyethylenoxid), Poly(phosphazenen), Polysulfonen, Lipiden, Polyacrylsäuren, Polymethylmethacrylat, Polyacrylamiden, Polyacrylonitrilen (Polycyanoacrylaten), Poly-HEMA, Polyurethanen, Polyolefinen, Polystyrol, Polyterephthalaten, Polyethylenen, Polypropylenen, Polyetherketonen, Polyvinylchloriden, Polyfluoriden, Silikonen, Polysilikaten (bioaktives Glas), Siloxanen (Polydimethylsiloxanen), Hydroxyapatiten, Lactidcapronolacton, natürlichen und nicht natürlichen Polyaminosäuren, Poly-β-aminoestern, Albuminen, Alginaten, Zellulose / Zelluloseacetaten, Chitin / Chitosan, Kollagen, Fibrin / Fibrinogen, Gelatine, Lignin, Polymeren auf Proteinbasis, Poly-(lysin), Poly(glutamat), Poly(malonaten), Poly(hyaluronsäuren), Polynukleinsäuren, Polysacchariden, Poly-(hydroxyalkanoaten), Polyisoprenoiden, Polymeren auf Stärkebasis, Copolymeren davon, linearen, verzweigten, hyperverzweigten Dendrimeren, vernetzten, funktionalisierten Derivaten davon oder Hydrogelen, die auf aktivierten Polyethylenglykolen basieren, die mit alkalischen hydrolysierten tierischen oder pflanzlichen Proteinen kombiniert sind.

13. Stent, Verwendung oder Kit nach einem der Ansprüche 1 bis 12, wobei mindestens ein Inhibitor in einer Mikrokaspel oder Nanokapsel eingekapselt ist.

14. Stent, Verwendung oder Kit nach Anspruch 13, wobei eine Nanokapsel eines von einem Poly(ethylenoxid)-Copolymer mit Poly(L-milchsäure) oder mit Poly(beta-benzyl-L-aspartat); einem Copolymer mit Poly(lactid-co-glycolid)-[(propylenoxid)-poly(ethylenoxide)]; Polyphosphazenderivaten; einem mit Poly(ethylenglycol) beschichteten Nanokügelchen; Poly(isobutylcyanoacrylat)-Nanokapseln Poly(gamma-benzyl-L- glutamat)/(Poly(ethylenoxid); Chitosan-Poly(ethylenoxid) Nanopartikeln; o-Carboxymethylatchitosan, oder einem festen lipiden Nanokügelchen ("solid lipid nanosphere" - SLN) umfasst.

15. Stent, Verwendung oder Kit nach Anspruch 13, wobei eine Mikrokapsel eine Mikrokapsel ist, die eines von Chitosan; einem beschichteten Alginat-Mikrokügelchen; einem N-(Aminoalkyl)chitosan-Mikrokügelchen; einem Chitosan/Kalziumalginat-Kügelchen, einem Poly(adipinsäureanhydrid)-Mikrokügelchen; einem Gellan-Gummi-Kügelchen; einem Poly(D,L-Lactid-co-glycolid)-Mikrokügelchen; einer Alginat-poly-L-lysin-Mikrokapsel; einem vernetzten Chitosan- Mikrokügelchen; einem Chitosan/Gelatine-Mikrokügelchen; einem vernetzten Chitosan-Netz-Kügelchen mit Abstandshaltergruppen; einem aliphatischen Polyester; einem 1,5-Diozepan-2-on-Mikrokügelchen; einem D,L-Dilactid-Mikrokügelchen; einem Triglycerid-Lipokügelchen; einem Polyelektrolyt-Komplex von Natriumalginatchitosan; einer Polypeptid-Mikrokapsel, Oran-Albumin-Mikrokügelchen, umfasst.

16. Stent, Verwendung oder Kit nach einem der Ansprüche 1 bis 15, wobei das Rhodamin 123 an ein löslich machendes Mittel gekoppelt ist.

17. Stent, Verwendung oder Kit nach Anspruch 16, wobei das löslich machende Mittel ein löslich machendes Mittel ist, das Cholesterol oder ein Derivat davon ist.

18. Stent, Verwendung oder Kit nach Anspruch 17, wobei die Cholesterolderivate beliebige von Cholesteryl-3-betahydroxybutyrat, Cholesteryl-halogeniertes Butyrat, Cholesteryl-halogeniertes Acetat, Cholesteryl-halogeniertes Acetoacetat, Cholesteryl-halogeniertes Acetamid, Cholesteryl-halogeniertes Crotonat, Cholesteryl-halogeniertes Aceton, Cholesteryl-halogeniertes Citrat oder Cholesteryl-halogeniertes Oleat sind.

19. Stent, Verwendung oder Kit nach Anspruch 17, wobei das löslich machende Mittel Vitamin A oder ein Derivat davon ist.

20. Stent, Verwendung oder Kit nach Anspruch 19, wobei das Derivat von Vitamin A Formel (IV) oder (V) ist: 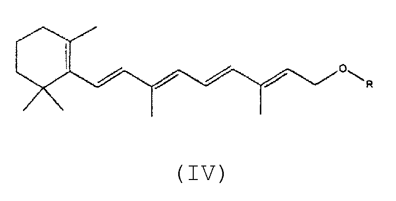 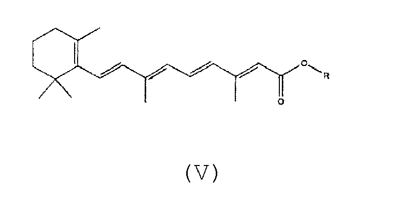 wobei R ausgewählt ist aus der Gruppe bestehend aus Betahydroxybutyrat, halogeniertem Butyrat, halogeniertem Acetat, halogeniertem Acetoacetat, halogeniertem Acetamid, halogeniertem Crotonat, halogeniertem Aceton, halogeniertem Citrat und halogeniertem Oleat.

21. Stent, Verwendung oder Kit nach einem der Ansprüche 1 bis 20, wobei das Rhodamin 123 in einer Menge vorhanden ist, um den oxidativen Phosphorylierungspfad zu hemmen.

22. Stent, Verwendung oder Kit nach einem der Ansprüche 1 bis 21, wobei der Stent mit einer Menge einer Zusammensetzung bereitgestellt ist, um eine Konzentration zwischen 10 und 60 mg Rhodamin 123/kg abzugeben.

23. Stent, Verwendung oder Kit nach einem der Ansprüche 1 bis 22, wobei die Zusammensetzung des Weiteren ein oder mehrere Polymere umfasst, um das Anheften der Zusammensetzung an den Stent und/oder die langsame Freisetzung der Zusammensetzung zu erleichtern.

24. Stent, Verwendung oder Kit nach Anspruch 23, wobei das Polymer eines oder mehr der Folgenden ist:
- aliphatische Polyester, Poly(aminosäuren), Copoly-(ether-ester), Polyalkylenoxalate, Polyamide, Poly-(iminokarbonate), Polyanhydride, Polyorthoester, Polyoxaester, Polyamidoester, Polymilchsäure, Polyethylenoxid, Polycaprolacton, Polyhydroxybutyratvalerate, Polyoxaester, die Amidogruppen enthalten, Poly(anhydride), Polyphosphazene, Silikone, Biomoleküle und Mischungen davon,
- Milchsäure D-,L- und Mesolactid, Epsilon-Caprolacton, Glycolidglycolsäure, Hydroxybutyrat, Hydroxyvalerat, para-Dioxanon, Trimethylencarbonat und seine Alkylderivate, 1,4-Dioxepan-2-on, 1,5-Dioxepan-2-on, 6,6-Dimethyl-1,4-dioxan-2-on und Polymermischungen davon,
- Polyanhydride von Disäuren der Form HOOC-C₆H₄-O-(CH₂)m-O-C₆H₄-COOH, wobei m eine ganze Zahl im Bereich von 2 bis 8 ist, und Copolymere davon mit aliphatischen Alpha-Omega-Disäuren von bis zu 12 Kohlenstoffen,
- natürlich enzymatisch oder hydrolytisch instabile Polymere, Fibrin, Fibrinogen, Kollagen, Gelatine, Glycosaminoglycane, Elastin und absorbierbare, biokompatible Polysaccharide, wie Chitosan, Stärke, Fettsäuren und Ester davon, Glucosoglycane und Hyaluronsäure,
- Hydrogele
- Polyurethane, Silikone, Poly(meth)acrylate, Polyester, Polyalkyloxide, Polyethylenoxid, Polyvinylalkohole, Polyethylenglycol und Polyvinylpyrrolidon, wie auch Hydrogele, Hydrogele von vernetztem Polyvinylpyrrolidinon und Polyestern, Polyolefine, Polyisobutylen und Ethylen-Alphaolefin-Copolymere, Acrylpolymere, Methacrylat und Copolymere, Vinylhalidpolymere und Copolymere, Polyvinylchlorid, Polyvinylether, Polyvinylmethylether, Polyvinyliden, Polyvinylidenfluorid, Polyvinylidenchlorid, Polyacrylonitril, Polyvinylketone, Polyvinylaromate, Polystyrol, Polyvinylester, Polyvinylacetat, Copolymere von Vinylmonomeren miteinander und mit Olefinen, Ethylen-Methylmethacrylat-Copolymere, Acrylonitril-Styrol-Copolymere, ABS-Harze und Ethylen-Vinylacetat-Copolymere, Polyamide, Nylon 66 und Polycaprolactam, Alkydharze, Polykarbonate, Polyoxymethylene, Polyimide, Polyether, Epoxidharze, Polyurethane, Rayon, Rayon-Triacetat, Zellulose, Zelluloseacetat, Zelluloseacetatbutyrat, Zellophan, Zellulosenitrat, Zellulosepropionat, Zelluloseether, Carboxymethylzellulose und Hydoxyalkylzellulosen und Kombinationen davon,
- Polyamide der Form NH-(CH₂)n-CO- und NH-(CH₂)x-NH-CO-(CH₂)y-CO, wobei n vorzugsweise eine ganze Zahl von 6 bis 13 ist; x eine ganze Zahl im Bereich von 6 bis 12 ist; und y eine ganze Zahl im Bereich von 4 bis 16 ist,
- biologisch absorbierbare Elastomere, aliphatische Polyesterelastomere,
- elastomere Copolymere von Epsilon-Caprolacton und Glycolid, vorzugsweise mit einem Molverhältnis von Epsilon-Caprolacton zu Glycolid von etwa 35:65 bis etwa 65:35, insbesondere 45:55 bis 35:65,
- elastomere Copolymere von Epsilon-Caprolacton und Lactid, L-Lactid, D-Lactid, Mischungen davon oder Milchsäure-Copolymere, vorzugsweise mit einem Molverhältnis von Epsilon-Caprolacton zu Lactid von etwa 35:65 bis etwa 90:10 und insbesondere von etwa 35:65 bis etwa 65:35 und ganz besonders von etwa 45:55 bis 30:70 oder von etwa 90:10 bis etwa 80:20,
- elastomere Copolymere von p-Dioxanon und Lactid, einschließlich L-Lactid, D-Lactid und Milchsäure, vorzugsweise mit einem Molverhältnis von p-Dioxanon zu Lactid von etwa 40:60 bis etwa 60:40,
- elastomere Copolymere von Epsilon-Caprolacton und p-Dioxanon, vorzugsweise mit einem Molverhältnis von Epsilon-Caprolacton zu p-Dioxanon von etwa 30:70 bis etwa 70:30,
- elastomere Copolymere von p-Dioxanon und Trimethylenkarbonat, vorzugsweise mit einem Molverhältnis von p-Dioxanon zu Trimethylenkarbonat von etwa 30:70 bis etwa 70:30,
- elastomere Copolymere von Trimethylenkarbonat und Glycolid, vorzugsweise mit einem Molverhältnis von Trimethylenkarbonat zu Glycolid von etwa 30:70 bis etwa 70:30,
- elastomeres Copolymer von Trimethylenkarbonat und Lactid einschließlich L-Lactid, D-Lactid, Mischungen davon oder Milchsäure-Copolymeren, vorzugsweise mit einem Molverhältnis von Trimethylenkarbonat zu Lactid von etwa 30:70 bis etwa 70:30, und Mischungen davon.

25. Stent oder Kit nach einem der Ansprüche 1 bis 24, geeignet zur Verwendung in der Hemmung der Zellproliferation.

26. Stent, Verwendung oder Kit nach einem der Ansprüche 1 bis 25, wobei die Zellproliferation Restenose oder Stenose ist.

27. Stent, Verwendung oder Kit nach einem der Ansprüche 1 bis 26, wobei die Zellproliferation Krebs ist.

28. Stent, Verwendung oder Kit nach einem der Ansprüche 1 bis 27, wobei der Stent in einer Arterie oder Vene angeordnet ist.

29. Stent, Verwendung oder Kit nach einem der Ansprüche 1 bis 27, wobei der Stent in einem Bronchialkanal angeordnet ist.

30. Stent, Verwendung oder Kit nach einem der Ansprüche 1 bis 27, wobei der Stent in einem Verdauungskanal, wie Speiseröhre, Darm und/oder Gallengang angeordnet ist.

31. Stent, Verwendung oder Kit nach einem der Ansprüche 1 bis 27, wobei der Stent in einem gynäkologischen Kanal, wie Uterus, Cervix und/oder Vagina angeordnet ist.

32. Stent, Verwendung oder Kit nach einem der Ansprüche 1 bis 27, wobei der Stent in einem Resektionshohlraum oder einer Narbe angeordnet ist.

33. Stent, Verwendung oder Kit nach einem der Ansprüche 1 bis 32, wobei das Rhodamin ein Derivat von Rhodamin ist.

34. Medizinischer Stent, der mindestens teilweise mit einer expandierbaren Membran über der expandierbaren Region bedeckt ist, wobei die Membran mit einer Zusammensetzung bereitgestellt ist, die Rhodamin 123 umfasst.

35. Medizinischer Stent nach Anspruch 34, wobei die Membran biologisch abbaubares oder nicht biologisch abbaubares Material ist.

36. Medizinischer Stent nach Anspruch 34 oder 35, wobei die Membran mindestens teilweise aus einem von aliphatischen Polyester-Copolymeren, elastomeren Copolymeren von Epsilon-Caprolacton und Glycolid, elastomeren Copolymeren von E-Caprolacton und Lactid, Copolymeren von E-Caprolacton und Milchsäure, elastomeren Copolymeren von p-Dioxanon und Lactid, elastomeren Copolymeren von p-Dioxanon und Milchsäure, elastomeren Copolymeren von E-Caprolacton und p-Dioxanon, elastomeren Copolymeren von p-Dioxanon und Trimethylenkarbonat, elastomeren Copolymeren von Trimethylenkarbonat und Glycolid, elastomeren Copolymeren von Trimethylenkarbonat und Lactid oder elastomeren Copolymeren von Trimethylenkarbonat und Milchsäure gebildet ist.

37. Medizinischer Stent nach einem der Ansprüche 34 bis 36, wobei der Stent mindestens teilweise aus einem Material hergestellt ist, das in situ biologisch abbaubar ist und mit der Zusammensetzung imprägniert ist.

38. Medizinischer Stent nach einem der Ansprüche 34 bis 37, wobei der Stent eine Legierung auf Magnesiumbasis umfasst.

39. Medizinischer Stent nach einem der Ansprüche 34 bis 38, wobei der Stent mindestens teilweise aus einem Material besteht, das nicht biologisch abbaubar ist.

40. Medizinischer Stent nach einem der Ansprüche 34 bis 39, wobei die Membran mindestens teilweise mit der Zusammensetzung bereitgestellt ist.

41. Verwendung einer Zusammensetzung, die Rhodamin 123 umfasst, in der Herstellung einer Zusammensetzung zur Bereitstellung eines medizinischen Stents, der mindestens teilweise mit einer expandierbaren Membran zur Hemmung der Zellproliferation bedeckt ist.

42. Verwendung einer Zusammensetzung, die Rhodamin 123 umfasst, in der Herstellung einer Zusammensetzung zur Bereitstellung eines medizinischen Stents, der mindestens teilweise mit einer expandierbaren Membran zur Sensibilisierung proliferierender Zellen gegenüber einer Strahlentherapie bedeckt ist.

43. Verwendung einer Zusammensetzung, die Rhodamin 123 umfasst, in der Herstellung einer Zusammensetzung zur Bereitstellung eines medizinischen Stents, der mindestens teilweise mit einer expandierbaren Membran zur Sensibilisierung proliferierender Zellen gegenüber einer Chemotherapie bedeckt ist.

44. Kit, umfassend a) mindestens einen medizinischen Stent, der mindestens teilweise mit einer expandierbaren Membran bedeckt ist und b) eine Zusammensetzung, die Rhodamin 123 umfasst.

45. Medizinischer Stent, Verwendung oder Kit nach einem der Ansprüche 34 bis 44, wobei die Zusammensetzung des Weiteren ein oder mehr langsame Freisetzungsmittel umfasst, um ein langsames Freisetzen des Inhibitors zu erleichtern.

46. Medizinischer Stent, Verwendung oder Kit nach Anspruch 45, wobei das langsame Freisetzungsmittel eines wie in Anspruch 12 definiert ist.

47. Medizinischer Stent, Verwendung oder Kit nach einem der Ansprüche 34 bis 46, wobei das Rhodamin 123 in einer Mikrokapsel oder Nanokapsel eingekapselt ist.

48. Medizinischer Stent, Verwendung oder Kit nach Anspruch 47, wobei eine Nanokapsel eine Nanokapsel wie in Anspruch 14 definiert ist.

49. Medizinischer Stent nach Anspruch 48, wobei eine Mikrokapsel eine Mikrokapsel wie in Anspruch 15 definiert ist.

50. Medizinischer Stent, Verwendung oder Kit nach einem der Ansprüche 34 bis 49, wobei das Rhodamin 123 an ein löslich machendes Mittel gekoppelt ist.

51. Medizinischer Stent, Verwendung oder Kit nach Anspruch 50, wobei das löslich machende Mittel ein löslich machendes Mittel wie in einem der Ansprüche 17 bis 20 definiert ist.

52. Medizinischer Stent, Verwendung oder Kit nach einem der Ansprüche 34 bis 51, wobei die Zusammensetzung des Weiteren ein oder mehr Polymere umfasst, um ein Anheften der Zusammensetzung an die Membran und/oder eine langsame Freisetzung der Zusammensetzung zu erleichtern.

53. Medizinischer Stent, Verwendung oder Kit nach Anspruch 52, wobei das Polymer ein oder mehrere der Polymere wie in Anspruch 23 definiert ist.

54. Medizinischer Stent oder Kit nach einem der Ansprüche 34 bis 40 und 44 bis 53, geeignet zur Verwendung bei der Hemmung einer Zellproliferation.

55. Medizinischer Stent oder Kit oder Verwendung nach einem der Ansprüche 41 bis 54, wobei die Zellproliferation Restenose oder Stenose ist.

56. Medizinischer Stent, Kit oder Verwendung nach einem der Ansprüche 41 bis 54, wobei die Zellproliferation Krebs ist.

57. Medizinischer Stent, Kit oder Verwendung nach einem der Ansprüche 34 bis 56, wobei der Stent in einer Arterie oder Vene angeordnet ist.

58. Medizinischer Stent, Kit oder Verwendung nach einem der Ansprüche 34 bis 56, wobei der Stent in einem Bronchialkanal angeordnet ist.

59. Medizinischer Stent, Kit oder Verwendung nach einem der Ansprüche 34 bis 56, wobei der Stent in einem Verdauungskanal, wie Speiseröhre, Darm und/oder Gallengang angeordnet ist.

60. Medizinischer Stent, Kit oder Verwendung nach einem der Ansprüche 34 bis 56, wobei der Stent in einem gynäkologischen Kanal, wie Uterus, Cervix und/oder Vagina angeordnet ist.

61. Medizinischer Stent, Kit oder Verwendung nach einem der Ansprüche 34 bis 56, wobei der Stent in einem gutartigen oder bösartigen Resektionshohlraum oder einer Narbe angeordnet ist.

62. Stent, Verwendung oder Kit nach einem der Ansprüche 34 bis 61, wobei das Rhodamin ein Derivat von Rhodamin ist.

## Revendications

1. Endoprothèse médicale fournie avec une composition comprenant un inhibiteur de phosphorylation oxydante qui est la rhodamine 123.

2. Kit comprenant a) au moins une endoprothèse médicale et b) une composition comprenant un inhibiteur de phosphorylation oxydante qui est la rhodamine 123.

3. Utilisation d'une composition comprenant un inhibiteur de phosphorylation oxydante qui est la rhodamine 123, pour la préparation d'une composition pour fournir une endoprothèse médicale pour inhiber une prolifération cellulaire via l'inhibition du chemin de phosphorylation oxydante.

4. Utilisation d'une composition comprenant un inhibiteur de phosphorylation oxydante qui est la rhodamine 123, pour la préparation d'une composition pour fournir une endoprothèse médicale pour sensibiliser des cellules proliférantes au traitement par radiothérapie.

5. Utilisation d'une composition comprenant un inhibiteur de phosphorylation oxydante qui est la rhodamine 123, pour la préparation d'une composition pour fournir une endoprothèse médicale pour sensibiliser des cellules proliférantes au traitement par chimiothérapie.

6. Endoprothèse, utilisation ou kit selon l'une quelconque des revendications 1 à 5, dans lesquels ladite endoprothèse est fournie avec une ou plusieurs cavités pour contenir et libérer ladite composition.

7. Endoprothèse, utilisation ou kit selon l'une quelconque des revendications 1 à 6, dans lesquels ladite endoprothèse est au moins partiellement fabriquée dans un matériau qui est biodégradable *in situ.*

8. Endoprothèse, utilisation ou kit selon l'une quelconque des revendications 1 à 7, dans lesquels ladite endoprothèse comprend un alliage à base de magnésium.

9. Endoprothèse, utilisation ou kit selon l'une quelconque des revendications 1 à 8, dans lesquels ladite endoprothèse est au moins partiellement fabriquée dans un matériau qui est non biodégradable *in situ* et est imprégné de ladite composition.

10. Endoprothèse, utilisation ou kit selon l'une quelconque des revendications 1 à 9, dans lesquels ladite endoprothèse est au moins partiellement fournie avec ladite composition.

11. Endoprothèse, utilisation ou kit selon l'une quelconque des revendications 1 à 10, dans lesquels ladite composition comprend en outre un ou plusieurs agents à libération prolongée pour faciliter la libération prolongée de l'inhibiteur.

12. Endoprothèse, utilisation ou kit selon l'une quelconque des revendications 1 à 11, dans lesquels ledit agent à libération prolongée est l'un quelconque parmi des alliages de magnésium, un acide poly(glycolique), un acide poly(lactique) ou en général des polymères à base d'acide glycolique ou lactique, des copolymères, des polycaprolactones et en général des alkanoates polyhydroxyles, des (acides alcanoïques) poly(hydroxy), du poly(éthylèneglycol), de l'alcool polyvinylique, des poly-(orthoesters), des poly(anhydrides), des poly(carbonates), des polyamides, des polyimides, des polyimines, des poly-(iminocarbonates), des poly(éthylènimines), des polydioxanes, un (oxyde polyéthylénique) de polyoxyéthylène, des poly(phosphazènes) des polysulphones, des lipides, des acides polyacryliques, un polyméthylméthacrylate, des polyacrylamides, des polyacrylonitriles (polycyanoacrylates), des polyHEMA, des polyuréthanes, des polyoléfines, du polystyrène, des polytéréphtalates, des polyéthylènes, des polypropylènes, des polyéthercétones, des polyvinylchlorures, des polyfluorures, des silicones, des polysilicates (verre biactif), des siloxanes (polydiméthylsiloxanes), des hydroxyapatites, des capronolactone lactides, des polyaminoacides naturels et non naturels, des poly-β-aminoesters, des albumines, des alginates, de la cellulose / des acétates de cellulose, de la chitine / du chitosane, du collagène, de la fibrine /du fibrinogène, de la gélatine, de la lignine, des polymères à base de protéines, de la poly(lysine), du poly(glutamate), des poly(malonates), des acides poly(hyaluroniques) des acides polynucléiques, des polysaccharides, des poly(hydroxyalkanoates), des polyisoprénoïdes, des polymères à base d'amidon, des copolymères de ceux-ci, des dendrimères linéaires, ramifiés, hyper-ramifiés, des dérivés réticulés, fonctionnalisés de ceux-ci, ou des hydrogels à base de polyéthylèneglycols activés combinés à des protéines animales ou végétales hydrolysées alcalines.

13. Endoprothèse, utilisation ou kit selon l'une quelconque des revendications 1 à 12, dans lesquels au moins un inhibiteur est encapsulé dans une microcapsule ou une nanocapsule.

14. Endoprothèse, utilisation ou kit selon la revendication 13, dans lesquels une nanocapsule comprend l'un quelconque parmi un (oxyde) poly(éthylène) copolymère avec un (acide) poly(L-lactique) ou avec un poly(bétabenzyl-L-aspartate) ; un copolymère avec un poly(lactide-co-glycolide)-[(propylènoxyde)-poly(éthylène oxyde)] ; des dérivés de polyphosphazène ; une nanosphère enduite de poly(éthylèneglycol) ; des nanocapsules poly(isobutylcyanoacrylate) de poly(gamma-benzyl-L-glutamate)/(poly-(éthylène oxyde) ; des nanoparticules de chitosane-poly(éthylène oxyde) ; du chitosane de o-carboxyméthylate ; ou une nanosphère lipidique solide (SLN).

15. Endoprothèse, utilisation ou kit selon la revendication 13, dans lesquels une microcapsule est une microcapsule comprenant l'un quelconque parmi du chitosane ; une microsphère d'alginate enduite ; une microsphère de chitosane N-(aminoalkyle) ; une bille d'alginate de chitosane/calcium ; une microsphère poly(adipique anhydride) ; une bille de gomme gellane ; une microsphère de poly(D, L-lactide-co-glycolide) ; une microcapsule d'alginate-poly-L-lysine ; une microsphère de chitosane réticulé ; une microsphère de chitosane/gélatine ; une bille en réseau de chitosane réticulé avec des groupes espaceurs ; un polyester aliphatique ; une microsphère de 1,5-diozépan-2-one ; une microsphère D,L-dilactide ; une liposphère triglycéride ; un complexe polyélectrolyte de chitosane d'alginate de sodium ; une microcapsule de polypeptide ; une microsphère d'albumine orane.

16. Endoprothèse, utilisation ou kit selon l'une quelconque des revendications 1 à 15, ladite rhodamine 123 étant couplée à un agent de solubilisation.

17. Endoprothèse, utilisation ou kit selon la revendication 16, dans lesquels ledit agent de solubilisation est un agent de solubilisation est le cholestérol ou un dérivé de celui-ci.

18. Endoprothèse, utilisation ou kit selon la revendication 17, dans lesquels lesdits dérivés de cholestérol sont l'un quelconque parmi le cholestéryl-3-bétahydroxybutyrate, le butyrate halogéné par cholestéryle, l'acétate halogéné par cholestéryle, l'acéto-acétate halogéné par cholestéryle, l'acétamide halogéné par cholestéryle, le crotonate halogéné par cholestéryle, l'acétone halogénée par cholestéryle, le citrate halogéné par cholestéryle, ou l'oléate halogéné par cholestéryle.

19. Endoprothèse, utilisation ou kit selon la revendication 17, dans lesquels ledit agent de solubilisation est la vitamine A ou un dérivé de celle-ci.

20. Endoprothèse, utilisation ou kit selon la revendication 19, dans lesquels le dérivé de vitamine A est la formule (IV) ou (V) : 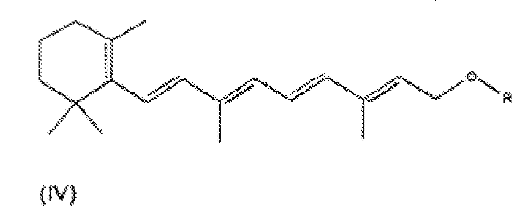 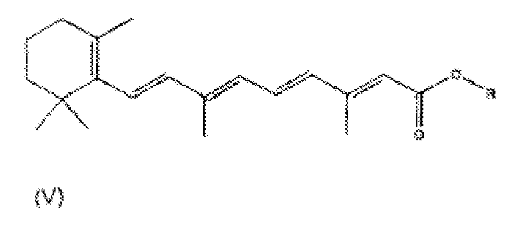 dans lesquelles R est sélectionné parmi le groupe constitué de bétahydroxybutyrate, butyrate halogéné, acétate halogéné, acéto-acétate halogéné, acétamide halogéné, crotonate halogéné, acétone halogénée, citrate halogéné, et oléate halogéné.

21. Endoprothèse, utilisation ou kit selon l'une quelconque des revendications 1 à 20, dans lesquels la rhodamine 123 est présente dans une quantité pour inhiber le chemin de phosphorylation oxydante.

22. Endoprothèse, utilisation ou kit selon l'une quelconque des revendications 1 à 21, dans lesquels ladite endoprothèse est fournie avec une quantité de composition pour livrer une concentration comprise entre 10 et 60 mg de rhodamine 123/kg.

23. Endoprothèse, utilisation ou kit selon l'une quelconque des revendications 1 à 22, dans lesquels ladite composition comprend en outre un ou plusieurs polymères pour faciliter l'attachement de la composition à l'endoprothèse et/ou la libération prolongée de la composition.

24. Endoprothèse, utilisation ou kit selon la revendication 23, dans lesquels ledit polymère est un ou plusieurs parmi:
- polyesters aliphatiques, poly(amino acides), copoly(éther-esters), polyalkylènes oxalates, polyamides, poly(iminocarbonates), polyanhydrides, polyorthoesters, polyoxaesters, polyamidoesters, acide polylactique, polyéthylènoxyde, polycaprolactone, valérates de polyhydroxybutyrate, polyoxaesters contenant des groupes amido, poly-(anhydrides), polyphosphazènes, silicones, biomolécules et des mélanges de ceux-ci,
- acide lactique, D-,L- et méso lactide, epsilon-caprolactone, acide glycolique de glycolide, hydroxybutyrate, hydroxyvalérate, paradioxanone, carbonate de triméthylène et ses dérivés alkyle, 1,4-dioxépan-2-one, 1,5-dioxépan-2-one, 6,6-diméthyl-1,4-dioxan-2-one et des mélanges de polymères de ceux-ci,
- polyanhydrides de diacides de la forme HOOC-C₆H₄-O-(CH)₂m-O-C₆H₄-COOH dans lesquels m est un entier dans la gamme de 2 à 8 et des copolymères de ceux-ci avec des diacides alpha-oméga aliphatiques avec jusqu'à 12 carbones,
- polymères enzymatiquement ou hydrolytiquement instables naturellement, fibrine, fibrinogène, collagène, gélatine, glycosaminoglycanes, élastine, et polysaccharides biocompatibles absorbables telles que chitosane, amidon, acides gras et esters de ceux-ci, glucoso-glycanes et acide hyaluronique,
- hydrogels,
- polyuréthanes, silicones, poly(méth)acrylates, polyesters, oxydes de polyalkyle, oxyde de polyéthylène, alcools polyvinyliques, polyéthylèneglycols et polyvinylpyrrolidone, ainsi que hydrogels, hydrogels de polyvinylpyrrolidone réticulée et polyesters, polyoléfines, copolymères de polyisobutylène et éthylène-alphaoléfines, polymères acryliques, méthacrylates et copolymères, polymères et copolymères d'halogénures de vinyle, chlorure de polyvinyle, éthers de polyvinyle, polyvinylméthyléther, polyvinylidène, fluorure de polyvinylidène, chlorure de polyvinylidène, polyacrylonitrile, polyvinylcétones, hydrocarbures aromatiques de polyvinyle, polystyrène, polyvinylesters, polyvinylacétate, copolymères de monomères de vinyle avec les uns les autres et des oléfines, copolymères de méthacrylate d'éthylène-méthyle, copolymères d'acrylonitrile-styrène, résines ABS et copolymères d'acétate d'éthylène-vinyle, polyamides, Nylon 66 et polycaprolactame, résines d'alkyde, polycarbonates, polyoxyméthylènes, polyimides, polyéthers, résines époxy, polyuréthanes, rayonne, rayonne-triacétate, cellulose, acétate de cellulose, butyrate d'acétate de cellulose, cellophane, nitrate de cellulose, propionate de cellulose, éthers de cellulose, carboxyméthylcellulose et hydroxyalkylcelluloses, et des combinaisons de ceux-ci,
- polyamides de la forme -NH-(CH₂)n-CO et NH-(CH₂)x-NH-CO-(CH₂)y-CO, dans lesquels n est de préférence un entier dans la gamme de 6 à 13 ; x est un entier dans la gamme de 6 à 12 ; et y est un entier dans la gamme de 4 à 16,
- élastomères bioabsorbables, élastomères de polyesters aliphatiques,
- copolymères élastomères d'epsilon-caprolactone et de glycolide, de préférence ayant un rapport molaire d'epsilon-caprolactone sur le glycolide d'environ 35 : 65 à environ 65 : 35, plus préférablement de 45 : 55 à 35 : 65,
- copolymères élastomères d'epsilon-caprolactone et lactide, L-lactide, mélanges de D-lactides de ceux-ci ou copolymères d'acide lactique, de préférence ayant un rapport molaire d'epsilon-caprolactone sur la lactide d'environ 35 : 65 à environ 90 : 10, et plus préférablement d'environ 35 : 65 à environ 65 : 35 et plus préférablement d'environ 45 : 55 à 30 : 70 ou d'environ 90 : 10 à environ 80 : 20,
- copolymères élastomères de p-dioxanone et lactide comprenant du L-lactide, D-lactide et de l'acide lactique, de préférence ayant un rapport molaire de p-dioxanone sur la lactide d'environ 40 : 60 à environ 60 : 40,
- copolymères élastomères d'epsilon-caprolactone et de p-dioxanone, de préférence ayant un rapport molaire d'epsilon-caprolactone sur p-dioxanone d'environ 30 : 70 à environ 70 : 30,
- copolymères élastomères de p-dioxanone et de triméthylènecarbonate, de préférence ayant un rapport molaire de p-dioxanone sur triméthylènecarbonate d'environ 30 : 70 à environ 70 : 30,
- copolymères élastomères de triméthylènecarbonate et de glycolide, de préférence ayant un rapport molaire de triméthylènecarbonate sur glycolide d'environ 30 : 70 à environ 70 : 30,
- copolymère élastomère de triméthylènecarbonate et lactide y compris L-lactide, D-lactide, des mélanges des ceux-ci ou des copolymères d'acide lactique, de préférence ayant un rapport molaire de triméthylènecarbonate sur lactide d'environ 30 : 70 à environ 70 : 30 et des mélanges de ceux-ci.

25. Endoprothèse ou kit selon l'une quelconque des revendications 1 à 24, approprié(e) pour une utilisation dans l'inhibition de la prolifération cellulaire.

26. Endoprothèse, utilisation ou kit selon l'une quelconque des revendications 1 à 25, dans lesquels ladite prolifération cellulaire est la resténose ou la sténose.

27. Endoprothèse, utilisation ou kit selon l'une quelconque des revendications 1 à 26, dans lesquels ladite prolifération cellulaire est le cancer.

28. Endoprothèse, utilisation ou kit selon l'une quelconque des revendications 1 à 27, dans lesquels l'endoprothèse est placée dans une artère ou une veine.

29. Endoprothèse, utilisation ou kit selon l'une quelconque des revendications 1 à 27, dans lesquels l'endoprothèse est placée dans un conduit bronchique.

30. Endoprothèse, utilisation ou kit selon l'une quelconque des revendications 1 à 27, dans lesquels l'endoprothèse est placée dans un conduit digestif tel que l'oesophage, l'intestin et/ou le tractus biliaire.

31. Endoprothèse, utilisation ou kit selon l'une quelconque des revendications 1 à 27, dans lesquels l'endoprothèse est placée dans un conduit gynécologique tel que l'utérus, le col de l'utérus et/ou le vagin.

32. Endoprothèse, utilisation ou kit selon l'une quelconque des revendications 1 à 27, dans lesquels l'endoprothèse est placée dans une cavité de résection ou une cicatrice.

33. Endoprothèse, utilisation ou kit selon l'une quelconque des revendications 1 à 32, dans lesquels ladite rhodamine est un dérivé de rhodamine.

34. Endoprothèse médicale au moins partiellement couverte d'une membrane extensible sur la région extensible, ladite membrane étant fournie avec une composition comprenant de la rhodamine 123.

35. Endoprothèse médicale selon la revendication 34, dans laquelle ladite membrane est en un matériau biodégradable ou non biodégradable.

36. Endoprothèse médicale selon la revendication 34 ou la revendication 35, dans laquelle ladite membrane est au moins partiellement formée à partir de l'un quelconque parmi des copolymères polyesters aliphatiques, copolymères élastomères d'epsilon-caprolactone et glycolide, copolymères élastomères d'E-caprolactone et lactide, copolymères d'E-caprolactone et acide lactique, copolymères élastomères de p-dioxanone et lactide, copolymères élastomères de p-dioxanone et acide lactique, copolymères élastomères d'epsilon-caprolactone et p-dioxanone, copolymères élastomères de p-dioxanone et triméthylènecarbonate, copolymères élastomères de triméthylènecarbonate et de glycolide, copolymère élastomère de triméthylènecarbonate et lactide, ou copolymère élastomère de triméthylènecarbonate et acide lactique.

37. Endoprothèse médicale selon l'une quelconque des revendications 34 à 36, dans laquelle ladite endoprothèse est au moins partiellement fabriquée à partir d'un matériau qui est biodégradable *in situ* et est imprégné de ladite composition.

38. Endoprothèse médicale selon l'une quelconque des revendications 34 à 37, dans laquelle ladite endoprothèse comprend un alliage à base de magnésium.

39. Endoprothèse médicale selon l'une quelconque des revendications 34 à 38, dans laquelle ladite endoprothèse est au moins partiellement fabriquée à partir d'un matériau qui est non biodégradable.

40. Endoprothèse médicale selon l'une quelconque des revendications 34 à 39, dans laquelle ladite endoprothèse est au moins partiellement fournie avec ladite composition.

41. Utilisation d'une composition comprenant de la rhodamine 123, pour la préparation d'une composition pour fournir une endoprothèse médicale au moins partiellement couverte d'une membrane extensible pour l'inhibition de la prolifération cellulaire.

42. Utilisation d'une composition comprenant de la rhodamine 123, pour la préparation d'une composition pour fournir une endoprothèse médicale au moins partiellement couverte d'une membrane extensible pour la sensibilisation de cellules proliférantes à la radiothérapie.

43. Utilisation d'une composition comprenant de la rhodamine 123, pour la préparation d'une composition pour fournir une endoprothèse médicale au moins partiellement couverte d'une membrane extensible pour la sensibilisation de cellules proliférantes à la chimiothérapie.

44. Kit comprenant a) au moins une endoprothèse médicale au moins partiellement couverte d'une membrane extensible et b) une composition comprenant de la rhodamine 123.

45. Endoprothèse, utilisation ou kit selon l'une quelconque des revendications 34 à 44, dans lesquels ladite composition comprend en outre un ou plusieurs agents à libération prolongée pour faciliter la libération prolongée de l'inhibiteur.

46. Endoprothèse, utilisation ou kit selon la revendication 45, dans lesquels l'agent à libération prolongé est l'un quelconque parmi ceux définis à la revendication 12.

47. Endoprothèse, utilisation ou kit selon l'une quelconque des revendications 34 à 46, dans lesquels ladite rhodamine 123 est encapsulée dans une microcapsule ou une nanocapsule.

48. Endoprothèse, utilisation ou kit selon la revendication 47, dans lesquels une nanocapsule est une nanocapsule telle que définie à la revendication 14.

49. Endoprothèse, utilisation ou kit selon la revendication 48, dans lesquels une microcapsule est une microcapsule telle que définie à la revendication 15.

50. Endoprothèse, utilisation ou kit selon l'une quelconque des revendications 34 à 49, dans lesquels ladite rhodamine 123 est couplée à un agent de solubilisation.

51. Endoprothèse, utilisation ou kit selon la revendication 50, dans lesquels ledit agent de solubilisation est un agent de solubilisation tel que défini dans l'une quelconque des revendications 17 à 20.

52. Endoprothèse, utilisation ou kit selon l'une quelconque des revendications 34 à 51, dans lesquels ladite composition comprend en outre un ou plusieurs polymères pour faciliter l'attachement de la composition à la membrane et/ou la libération prolongée de la composition.

53. Endoprothèse, utilisation ou kit selon la revendication 52, dans lesquels ledit polymère est un ou plusieurs parmi les polymères tels que définis à la revendication 23.

54. Endoprothèse ou kit selon l'une quelconque des revendications 34 à 40 et 44 à 53, approprié(e)s pour une utilisation dans l'inhibition de la prolifération cellulaire.

55. Endoprothèse, kit ou utilisation selon l'une quelconque des revendications 41 à 54, dans lesquels ladite prolifération cellulaire est la resténose ou la sténose.

56. Endoprothèse, kit ou utilisation selon l'une quelconque des revendications 41 à 54, dans lesquels ladite prolifération cellulaire est le cancer.

57. Endoprothèse, kit ou utilisation selon l'une quelconque des revendications 34 à 56, dans lesquels ladite endoprothèse est placée dans une artère ou une veine.

58. Endoprothèse, kit ou utilisation selon l'une quelconque des revendications 34 à 56, dans lesquels ladite endoprothèse est placée dans un conduit bronchique.

59. Endoprothèse, kit ou utilisation selon l'une quelconque des revendications 34 à 56, dans lesquels ladite endoprothèse est placée dans un conduit digestif tel que l'oesophage, l'intestin, et/ou le tractus biliaire.

60. Endoprothèse, kit ou utilisation selon l'une quelconque des revendications 34 à 56, dans lesquels ladite endoprothèse est placée dans un conduit gynécologique tel que l'utérus, le col de l'utérus, et/ou le vagin.

61. Endoprothèse, kit ou utilisation selon l'une quelconque des revendications 34 à 56, dans lesquels ladite endoprothèse est placée dans une cavité de résection ou une cicatrice maligne ou bénigne.

62. Endoprothèse, kit ou utilisation selon l'une quelconque des revendications 34 à 61, dans lesquels ladite rhodamine est un dérivé de la rhodamine.
